# EUROPEAN PATENT SPECIFICATION

(11) **EP 1 105 154 B1**
(45) Date of publication and mention of the grant of the patent: **01.06.2016**
(21) Application number: 98940866.1
(22) Date of filing: 13.08.1998
(51) Int. Cl.: A61K 39/00, A61K 39/44, A61K 39/02, A61K 39/085, C12N 15/63, C12N 1/20, C12N 1/21, C12N 15/00, C12P 21/00, G01N 33/53, C07K 14/00, C07K 14/31

(54) **BACTERIAL SUPERANTIGEN VACCINES**
IMPFSTOFFE GEGEN BAKTERIELLE SUPERANTIGENE
VACCINS À SUPERANTIGÈNES BACTÉRIENS

(43) Date of publication of application: 13.06.2001
(73) Proprietor: Walter Reed Army Institute of Research, Washington, DC 20307 (US)
(72) Inventor: ULRICH, Robert, G., Frederick, MD 21702 (US); OLSON, Mark, A., Gaithersburg, MD 20877 (US); BAVARI, Sina, Dillsburg, PA 17019 (US)
(74) Representative: Lord, Hilton David
(86) International application number: PCT/US1998/016766
(87) International publication number: WO 2000/009154

(56) References cited:
- WO-A-96/36366
- WO-A-97/36932
- WO-A2-00/02523
- BRIGGS C ET AL: "Mutations affecting the superantigen activity of staphylococcal enterotoxin B" IMMUNOLOGY, vol. 90, no. 2, 1997, pages 169-175, XP008036301 ISSN: 0019-2805
- ROSENDAHL ALEXANDER ET AL: "A mutation of F47 to A in staphylococcus enterotoxin A activates the T-cell receptor Vbeta repertoire in vivo" INFECTION AND IMMUNITY, vol. 65, no. 12, December 1997 (1997-12), pages 5118-5124, XP002298922 ISSN: 0019-9567
- GROSSMAN D ET AL: "MUTATION OF THE DISULFIDE LOOP IN STAPHULOCOCCAL ENTEROTOXIN A CONSEQUENCES FOR T CELL RECOGNITION" JOURNAL OF IMMUNOLOGY, THE WILLIAMS AND WILKINS CO. BALTIMORE, US, vol. 147, no. 10, 15 November 1991 (1991-11-15), pages 3274-3281, XP000644725 ISSN: 0022-1767
- DANNECKER G. ET AL: 'Activation of Human T Cells by the Superantigen Staphylococcus Enterotoxin B: Analaysis on a Cellular Level' IMMUNOBIOLOGY vol. 190, 1994, USA, pages 116 - 126, XP002914963
- GONZALO J.A. ET AL: 'Expansion and Clonal Deletion of Peripheral T Cells Induced by Bacterial Superantigen is Independent of the Interleukin-2 Pathway' EUR. J. IMMUNOL. vol. 22, 1992, pages 1007 - 1011, XP002914966
- BAVARI S. ET AL: 'Genetically Attenuated Bacterial Superantigen Vaccines' IMMUNOLOGY AND MOLECULAR BIOLOGY vol. SUPP.21A, no. C2-204, February 1995, page 88, XP002914967
- BAVARI S. ET AL: 'Staphalococcal Enterotoxin A and Toxic Shock Syndrome Toxin Compete with CD4 for Human Major Histocompatibility Complex Class II Binding' INFECTION AND IMMUNITY vol. 63, no. 2, February 1995, USA, pages 423 - 429, XP002914961
- MAHANA W. ET AL: 'A Natural Mutation of the Amino Acid Residue at Position 60 Destroys Staphylococcal Enterotoxin A Murine T-Cell Mitogenicity' INFECTION AND IMMUNITY vol. 63, no. 8, August 1995, USA, pages 2826 - 2832, XP002914962
- ULRICH R.G. ET AL: 'Bacterial Superantigens in Human Disease: Structure, Function and Diversity' TRENDS IN MICROBIOLOGY vol. 3, no. 12, 1995, pages 463 - 468, XP002914964
- WOODY M.A. ET AL: 'Differential Immune Responses to Staphylococcal Enterotoxin B Mutations in a Hydrophobic Loop Dominating the Interface with Major Histocompability Complex Class II Receptors' THE JOURNAL OF INFECTIOUS DISEASES vol. 177, 1998, pages 1013 - 1022, XP002914965
- HOLZER U ET AL: "T-cell stimulation and cytokine release induced by staphylococcal enterotoxin A (SEA) and the SEAD227A mutant", IMMUNOLOGY, vol. 90, no. 1, 1997, pages 74-80, ISSN: 0019-2805
- BAVARI S ET AL: "SUPERANTIGEN VACCINES: A COMPARATIVE STUDY OF GENETICALLY ATTENUATED RECEPTOR-BINDING MUTANTS OF STAPHYLOCOCCAL ENTEROTOXIN A", JOURNAL OF INFECTIOUS DISEASES. JID, UNIVERSITY OF CHICAGO PRESS, CHICAGO, IL, vol. 174, no. 2, 1 January 1996 (1996-01-01), pages 338-345, XP000916129, ISSN: 0022-1899

## Description

### INTRODUCTION

Staphylococcal enterotoxins (SEs) A through E are the most commom cause of food poisoning [Bergdoll, M.S.(1983) In Easom CSF, Aslam C., eds. Staphylococci and staphylococcal infections. London: Academic Press, pp 559-598] and are associated with several serious diseases [Schlievert, P.M. (1993) J. Infect. Dis. 167: 997-1002; Ulrich et al. (1995) Trends Microbiol. 3: 463-468], such as bacterial arthritis [Schwab et al. (1993) J. Immunol. 150; 4151-4159; Goldenberg et al. (1985) N. Engl. J. Med. 312: 764-771], other autoimmune disorders [Psnett, D. N. (1993) Semin. Immunol. 5: 65-72], and toxic shock syndrome [Schlieverst, P.M. (1986) Lancet 1: 1149-1150; Bohach et al. (1990) Crit. Rev. Microbiol. 17: 251-272]. The nonenterotoxic staphylococcal superantigen toxic shock syndrome toxin-1 (TSST-1) was first identified as a causative agent of menstrual-associated toxic shock syndrome [Schlievert et al. (1981) J. Infect. Dis. 143: 509-516]. Superantigen-producing *Staphylococcus aureus* strains are also linked to Kawasaki syndrome, an inflammatory disease of children [Leung et al. (1993) Lancet 342: 1385-1388].

The staphylococcal enterotoxins A-E, toxic shock syndrome toxin-1 (TSST-1), and streptococcal pyrogenic exotoxins A-C are soluble 23-29-kD proteins commonly referred to as bacterial superantigens (SAgs). Bacterial superantigens are ligands for both major histocompatibility complex (MHC) class II molecules, expressed on antigen-presenting cells, and the variable portion of the T cell antigen receptor **b** chain (TCR Vb) [Choi et al. (1989) Proc. Natl. Acad. Sci. USA 86:8941-8945; Fraser, J.D. (1989) Nature 339:221-223; Marrack et al. (1990) Science 248: 705-711; Herman et al. (1991) Annu. Rev. Immunol. 9: 745-772; Mollick et al. (1989) Science 244:817-820].

Each bacterial superantigen has a distinct affinity to a set of TCR Vb, and coligation of the MHC class II molecule polyclonally stimulates T cells [White et al. (1989) Cell 56: 27-35; Kappler et al. (1989) Science 244: 811-813; Takimoto et al. (1990) Eur J. Immunol. 140: 617-621]. Pathologically elevated levels of cytokines that are produced by activated T cells are the probable cause of toxic shock symptoms [Calson et al. (1985) Cell. Immunol. 96: 175-183; Stiles et al. (1993) Infect. Immun. 61: 5333-5338]. In addition, susceptibility to lethal gram-negative endotoxin shock is enhanced by several bacterial superantigens [Stiles, *et al., supra].* Although antibodies reactive with superantigens are present at low levels in human sera [Takei et al. (1993) J. Clin. Invest. 91: 602-607], boosting antibody titers by specific immunization may be efficacious for patients at risk for toxic shock syndrome and the other disorders of common etiology. A vaccine approach to controlling bacterial superantigen-associated diseases presents a unique set of challenges. Acute exposure to bacterial superantigens produces T cell anergy, a state of specific non-responsiveness [Kawabe et al. (1991) Nature 349: 245-248], yet T cell help is presumably a requirement for mounting an antibody response.

Bavari et al. (Journal of Infectious Diseases; 1996; 174:338-345) discloses site directed mutants of staphylococcal enterotoxin A that have attenuated binding to either T cell antigen receptors or MHC class II molecules.

WO00/02523 discloses compositions and methods for use in inducing an immune response using nucleic acids encoding mutant SEA and SEB exotoxins from staphylococcus aureus, which is protective against staphylococcal aureus intoxication in subjects.

Presently, the only superantigen vaccines available are chemically inactivated toxoids from different bacteria which have several disadvantages. The chemical inactivation process can be variable for each production lot making the product difficult to characterize. The chemical used for inactivation, (e.g. formaldehyde), is often toxic and does not negate the possibility of reversion of the inactivated superantigen to an active form. In addition, the yields of wild-type toxin from bacterial strains used for making toxoids are often low.

### SUMMARY OF THE INVENTION

The present invention relates to a vaccine which overcomes the disadvantages of the chemically inactivated toxoids described above. The superantigen vaccine(s) of the present invention is/are designed to protect individuals against the pathologies resulting from exposure to one or several related staphylococcal and streptococcal toxins. The superantigen vaccine is comprised of a purified protein product that is genetically attenuated by DNA methodologies such that superantigen attributes are absent, however the superantigen is effectively recognized by the immune system and an appropriate antibody response is produced.

Specifically, the vaccine of the present invention is a product of site-directed mutagenesis of the DNA coding sequences of superantigen toxins resulting in a disruption of binding to both the MHC class II receptor and to the T-cell antigen receptor. A comprehensive study of the relationships of the superantigen structures of TSST-1, streptococcal pyrogenic exotoxin-A (SPEa), staphylococcal enterotoxin B (SEB), and staphylococcal enterotoxin A, to receptor binding were undertaken to provide insight into the design of the vaccine. From these studies, critical amino acid residues of the toxin responsible for binding the superantigen to the human MHC receptor were defined. Site-directed mutagenesis of the gene encoding the toxin and expression of the new protein product resulted in a superantigen toxin with disrupted binding to the MHC receptors.

Therefore, it is an object of the present invention to provide an isolated and purified DNA fragment which encodes an altered Staphylococcal enterotoxin A (SEA) superantigen toxin peptide wherein position 64 has been change to alanine and one or more of positions 92, 70 and 48 has been changed to alanine, arginine, and/or arginine, respectively, whereby binding of the encoded altered toxin peptide to either the MHC class II or T-cell antigen receptor is altered. Such a DNA fragment is useful in the production of a vaccine against superantigen toxin infections.

It is another object of the present invention to provide an isolated and purified altered SEA superantigen toxin peptide which has been altered such that position 64 has been changed to alanine and one or more of positions 92, 70 and 48 have been changed to alanine, arginine, and/or arginine, respectively, whereby binding of the encoded altered toxin to the MHC class II or T-cell antigen receptor is altered. Such a sequence is useful for the production of a superantigen toxin vaccine.

It is another object of the invention to provide a recombinant vector comprising a vector and the DNA fragment described above.

It is a further object of the present invention to provide host cells transformed with the above-described recombinant DNA constructs. Host cells include cells of other prokaryotic species or eukaryotic plant or animal species, including yeasts, fungi, plant culture, mammalian and nonmammalian cell lines, insect cells and transgenic plants or animals.

It is another object of the present invention to provide a method for producing altered superantigen toxin with altered MHC class II and T-cell antigen receptor binding which comprises culturing a host cell under conditions such that a recombinant vector comprising a vector and the DNA fragment described above is expressed and altered superantigen toxin is thereby produced, and isolating superantigen toxin for use as a vaccine against superantigen toxin-associated bacterial infections and as a diagnostic reagent.

It is still another object of the invention to provide a purified altered superantigen toxin useful as a vaccine and as a diagnostic agent.

It is another object of the invention to provide a purified altered superantigen toxin for the therapeutic stimulation of, or other *in vivo* manipulation of, selective T cell subsets, or ex *vivo* manipulation of T cells for *in vivo* therapeutic purposes in mammals. Diseases, such as autoimmunity, wherein T-cell responses of limited diversity (oligoclonal) are evident. Altered superantigens may be used to therapeutically inactivate (induce anergy in) T cells in diseases wherein oligoclonal T-cell responses are evident such as autoimmune diseases, for example. For diseases in which specific T-cell subsets are not active or are anergetic, altered superantigens may be used to therapeutically stimulate these T cells. Such disease include, but are not limited to, infectious diseases and cancers wherein specific subsets of cytotoxic or helper T cells are inactivated or are otherwise unable to respond normally to the antigenic stimulation of the disease moiety.

There is also disclosed an antibody to the above-described altered superantigen toxin for use as a therapeutic agent and as a diagnostic agent.

It is yet another object of the invention to provide a superantigen toxin vaccine comprising an altered superantigen toxin peptide as described above, effective for the production of antigenic and immunogenic response resulting in the protection of a mammal against superantigen-associated bacterial infection.

It is a further object of the invention to provide a multivalent superantigen toxin vaccine comprising altered superantigen toxins from the group consisting of SEB and SEA effective for the production of antigenic and immunogenic response resulting in the protection of animal mammal against superantigen-associated bacterial infection.

It is yet another object of the present invention to provide a method for the diagnosis of superantigen toxin-associated bacterial infection comprising the steps of:
(i) contacting a sample from an individual suspected of having a superantigen toxin-associated bacterial infection with altered superantigen toxin peptide as described above; and
(ii) detecting the presence or absence of a superantigen-associated bacterial infection by detecting the presence or absence of a complex formed between superantigen toxin peptide and antibodies specific therefor in the sample.

There is disclosed a method for the diagnosis of superantigen bacterial infection comprising the steps of:
(i) contacting a sample from an individual suspected of having the disease with lymphocytes which recognize superantigen toxin produced by said superantigen bacteria or lymphocytes which recognize altered superantigen toxin; and (ii) detecting the presence or absence of responses of lymphocytes resulting from recognition of superantigen toxin. Responses can be, for example, measured cytokine release, increase of activation markers, mitotic activity, or cell lysis. The lymphocytes responding to the altered superantigen toxins recognize them as recall antigens not as superantigens, therefore the response is an indicator of prior exposure to the specific superantigen. The absence of a response may indicate no prior exposure, a defective immune response or in some cases a manifestation of T-cell anergy. Anergy is defined here as antigen-specific or a generalized non-responsiveness of subsets of T cells.

It is a further object of the present invention to provide a superantigen toxin-associated infection diagnostic kit comprising an altered superantigen toxin peptide as described above and ancillary reagents suitable for use in detecting the presence or absence of antibodies against superantigen toxin in a mammalian sample.

There is disclosed a detection method for detecting superantigen toxins or antibodies to superantigen toxin in samples, said method comprising employing a biosensor approach. Such methods are known in the art and are described for example in Karlsson et al. (1991) J. Immunol. Methods 145, 229-240 and Jonsson et al. (1991) Biotechniques 11, 620-627.

There is disclosed a therapeutic method for the treatment or amelioration of symptoms of
superantigen-associated bacterial infection, said method comprising providing to an individual in need of such treatment an effective amount of sera from individuals immunized with one or more altered superantigen toxins from different bacteria in a pharmaceutically acceptable excipient.

There is also disclosed a therapeutic method for the treatment or amelioration of symptoms of superantigen toxin-associated bacterial infection, said method comprising providing to an individual in need of such treatment an effective amount of antibodies against altered superantigen toxins in a pharmaceutically acceptable excipient.

There is disclosed a therapeutic method for the treatment or amelioration of symptoms of bacterial superantigen toxin infection, said method comprising providing to an individual in need of such treatment an effective amount of altered superantigen from a variety of streptococcal and staphylococcal bacteria in order to inhibit adhesion of superantigen bacterial toxin to MHC class II or T-cell receptors by competitive inhibition of these interactions.

There is disclosed a therapeutic method for the treatment of diseases that may not be associated directly with superantigen toxins but which result in specific nonresponsiveness of T-cell subsets, said method comprising the administration of altered superantigen toxins, *in vivo* or *ex vivo,* such that T-cell subsets are expanded or stimulated. Diseases which cause anergy or nonresponsiveness of T-cells include, but are not limited to, infectious diseases.

There is disclosed a therapeutic method for the treatment of diseases associated with expanded or over-stimulated T-cell subsets, such as autoimmunity for example, said method comprising administration of altered superantigen toxin, *in vivo* or *ex vivo,* such that anergy or inactivation of disease associated T-cells is produced. In this case, superantigen mutants can be designed with altered but not attenuated T-cell receptor binding, to cause anergy of only the select (i.e. 1-3) T-cell subsets that are pathologically activated.

### BRIEF DESCRIPTION OF THE DRAWINGS

These and other features, aspects, and advantages of the present invention will become better understood with reference to the following description and appended claims, and accompanying drawings where:
**Figure 1****.** Staphylococcal and streptococcal superantigen amino acid sequence homologies, compiled with Genetics Computers Group of Univ. of Wisconsin software.
**Figure 2****.** Comparison of mutant SEB and SEA biological activities.
   **A.** SEB mutant HLA-DR1-binding; **B.** SEA mutant HLA-DR1-binding; **C.** T-cell recognition of SEA and SEB mutants. Binding of bacterial superantigens to cell surface DR1 was measured by laser fluorescence-activated flow cytometry. A representative experiment of three performed is shown. The mutants SEA D197N, the homologous SEB D199N, and SEA L11Y had no effect on binding or T-cell recognition, and were used for controls. Human T-cell proliferation, assessed by [³H]thymidine incorporation, was measured in response to SEA (Y64A) or SEB (Y61A) mutants and controls that retained DR1-binding affinities. Each data point represents the mean of triplicate determinations; SEM <5%.

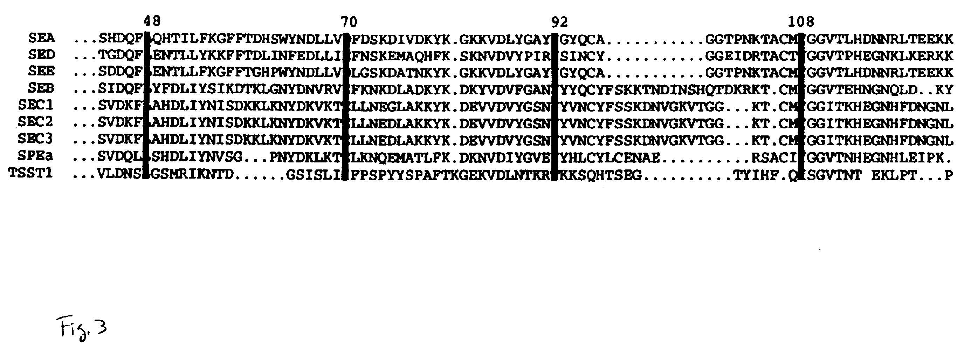

**Figure 3****.** Sequence and secondary structural alignment of bacterial superantigen toxins. Analyses were performed with the applications PILEUP and PROFILE from the Computer Genetics Group (Madison, WI) using sequence data obtained from a variety of sources. Amino acid residue numbering is based on the SEA sequence.

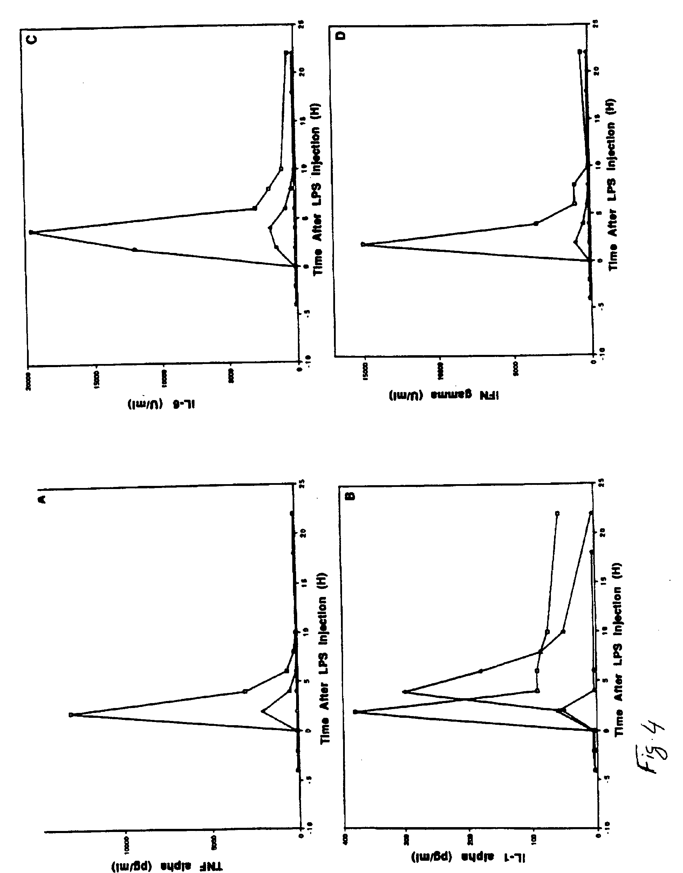

**Figure 4****.** Detection of TNF-α (a), IL-1α (B), IL-6 (C) and IFN-γ (D) in the serum of mice injected with SEA (open circles), LPS (open triangles), or SEA plus LPS (open squares). Values for TNF-α and IL-1α represent the mean of duplicate samples, with an SEM of ± 5%. INF-γ and IL-6 values represent the mean of duplicate and triplicate samples, respectively. The SEMs for IFN-γ and IL-6 readings were ± 5% and ± 10%, respectively.

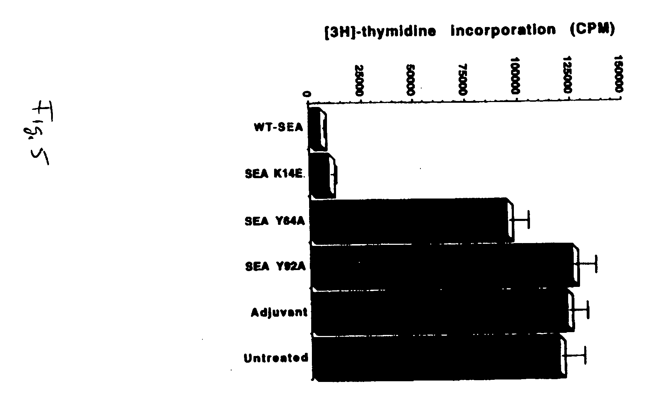

**Figure 5****.** Mutant SEA vaccines that have attenuated major histocompatibility complex class II or T-cell antigen receptor binding do not induce T-cell anergy. Mice were given three doses of wild type (WT) SEA or site-specific mutant vaccine, plus adjuvant. Control animals received adjuvant alone or were untreated; 2 weeks after final injection, pooled mononuclear cells were collected from spleens of 4 mice from each group. Results are represented as mean cpm (±SD) of quadruplicate wells incubated with 100 ng/ml WT SEA for 72 h and then pulse-labeled for 12 h with [³H]thymidine. P<0.0001 (analysis of variance for repeated measures comparing untreated, adjuvant, Y64A, and Y92A to WT SEA group).

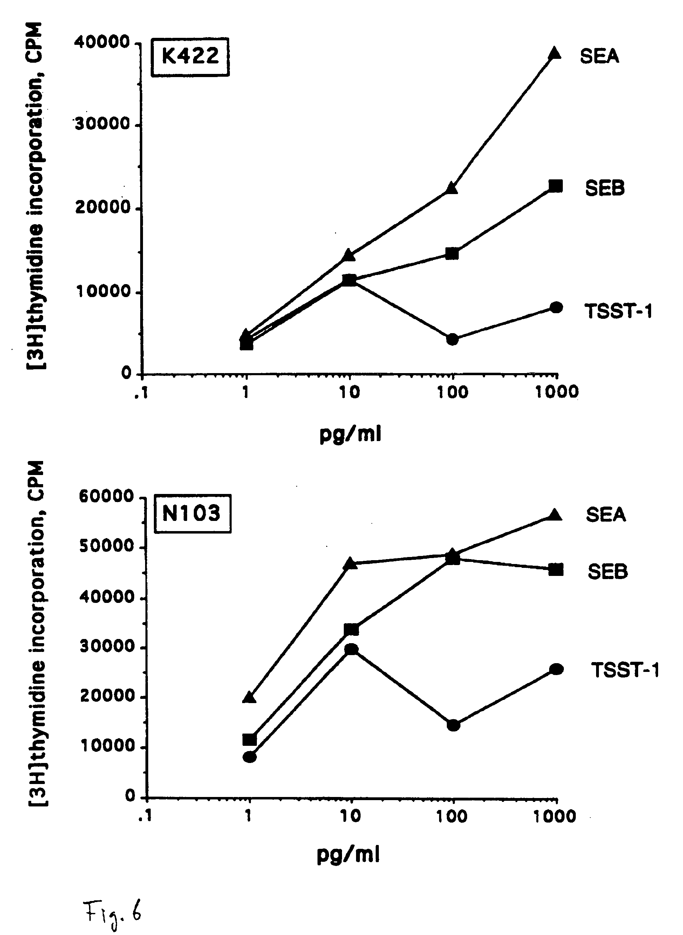

**Figure 6****.** No superantigen-induced T-cell anergy is exhibited by rhesus monkeys immunized with the vaccine B899445. Peripheral blood lymphocytes were incubated with titrated concentrations of wild-type superantigens from individual rhesus monkeys (K422 and N103) that were immunized with B899445. T-cell proliferation was assessed by [³H]thymidine incorporation. Each data point represents the mean of triplicate determinations; SEM <5%.

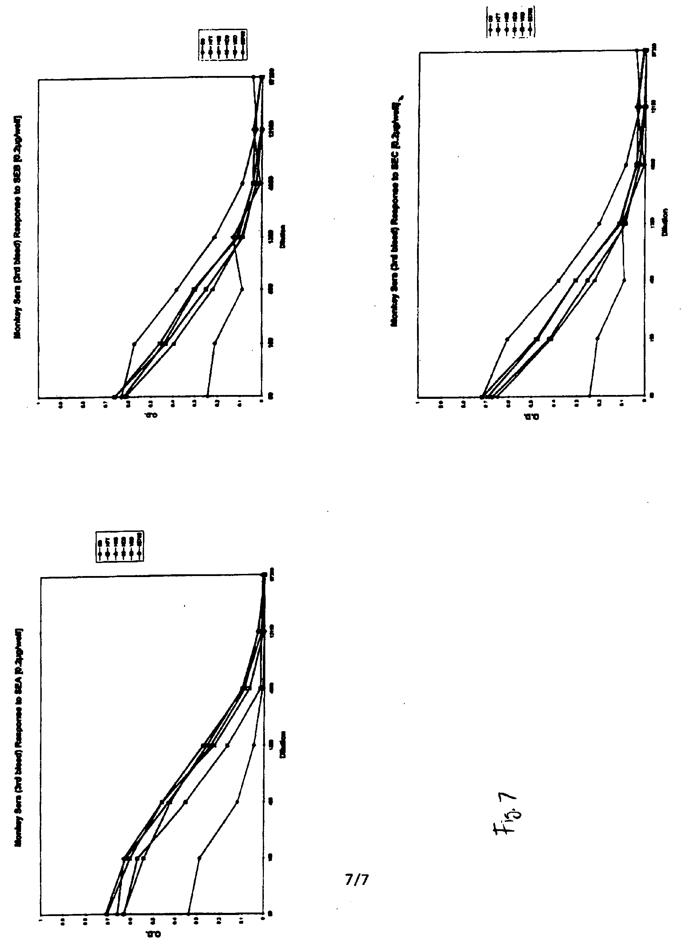

**Figure** 7. Antibody responses of rhesus monkeys immunized with a combined vaccine consisting of B899445 (SEB) and A489270 (SEA). The antibody levels were measured by ELISA, using plates coated with SEA, SEB or SEC1 as listed. Monkey G8 is a non-immunized control. SEM <5% for triplicate measurements.

### DETAILED DESCRIPTION

The present invention relates in part to a vaccine against superantigen toxin-associated bacterial diseases. The superantigen vaccines used in this study were developed by engineering changes in the receptor-binding portions of superantigen toxins to reduce receptor-binding affinities and toxicity while maintaining antigenicity.

Five different superantigen vaccines are described in this application: staphylococcal enterotoxin A, staplylococcal enterotoxin B, staphylococcal enterotoxin C1, toxic-shock syndrome toxin-1, and streptococcal pyrogenic exotoxin-A. For each of the superantigen toxins above, a comprehensive study of the relationships of the toxin protein structure to receptor binding was undertaken to provide insight into the design of the vaccine. The study employed site-directed mutagenesis of toxin and receptor molecules, molecular modeling, protein structure and binding studies. Following these studies, toxins were altered by site-directed mutagenesis to attenuate MHC class II binding and biological activity to an essentially non-specific level. The engineered vaccines were evaluated at each stage of analysis to determine mouse and human T-cell reactivities *in vitro,* serological responses and toxicity in mice and monkeys.

In one embodiment, the present invention relates to an altered superantigen toxin protein having an amino acid sequence which has been altered such that the binding of the toxin to the MHC class II receptor is disrupted.

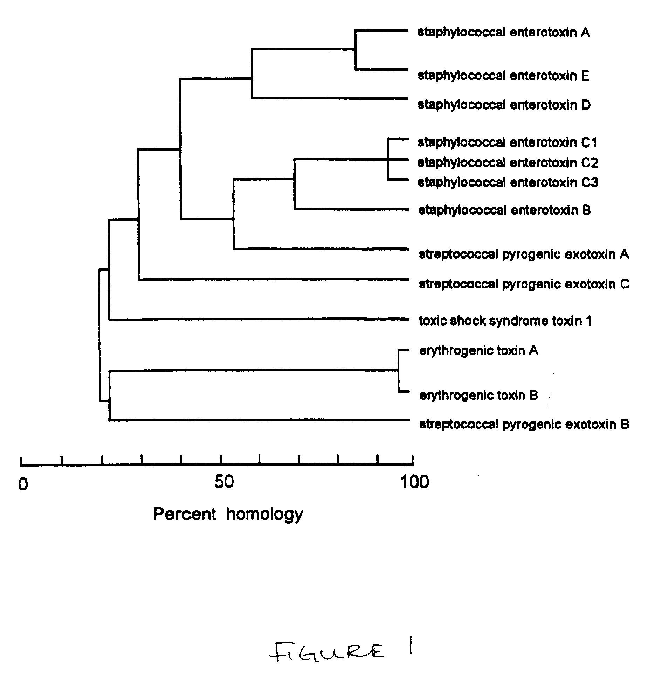

Comparison of amino acid sequences (**Fig. 1**) suggested that bacterial superantigens fall into groups consisting of (1) SEA, SED and SEE, (2) SEB, staphylococcal enterotoxins C1-C3 (SEC1-3), the streptococcal pyrogenic exotoxins A (SPE-A) and C (SPE-C), (3) TSST-1 and (4) the exfoliative toxins (ETA, ETB) and streptococcal pyrogenic exotoxin B (SPE-B), which are the most distant from the others in sequence. Although not available to the inventor when the inventions were first conceived and proof of principle was obtained, the x-ray crystallographic structures of several bacterial superantigens are now known. Diverse superantigens, such as SEB and TSST-1, appear to have little sequence in common, yet they exhibit homologous protein folds composed largely of β strands [Prasad, G.S. et al. (1993) Biochemistry 32, 13761-13766; Acharya, R.K. et al. (1994) Nature 367, 94-97; Swaminathan, S. et al. (1992) Nature 359, 801-806] within two distinct domains. Differences between the proteins are located primarily in highly variable regions comprised of several surface loops, such as the disulfide-bonded loop which is absent from TSST-1 and at the amino terminus.

The X-ray crystal structures of SEB and TSST-1 complexed with HLA DR1 are known[Kim, J. et al. (1994) Science 266, 1870-1874 ; Jardetzky, T.S. et al. (1994) Nature 368, 711-718]. The region of HLA DR1 that contacts SEB consists exclusively of α subunit surfaces. The main regions of SEB involved are two conserved sites: a polar pocket derived from three β strands of the β barrel domain and a highly solvent-exposed hydrophobic reverse turn. The polar binding pocket of SEB contains a glutamate and two tyrosines that accommodate Lys39 of the α subunit of HLA DR1, while the hydrophobic region consists of a leucine and flanking residues that make several contacts with the HLA DRα chain. The HLA DR1 binding sites of both TSST-1 and SEB overlap significantly. The hydrophobic binding contacts of other SAg with the HLA DRα chain have been proposed [Ulrich, et al. (1995). Nature, Struct. Biol 2, 554-560] to be similar to those found in SEB and TSST-1. A motif consisting of a leucine in a reverse turn [Ulrich *et al.* (1995), *supra]* is conserved among bacterial superantigens and may provide the key determinant (hydrophobic or otherwise) for binding HLA-DR. However, TSST-1 does not have a highly charged residue in the polar pocket that interacts with Lys39 of the HLA DRα chain and uses an alternative conformational binding mode that allows TSST-1 to interact with HLA DR1β-chain residues and the carboxy-terminal region of the antigenic peptide.

Both SEA and SEE bind to the β subunit of DR by means of a single zinc atom [Fraser, J.D. et al. (1992) Proc. Natl. Acad. Sci. USA 89, 5507-5511]. The amino-terminal domain of SEA interfaces with the HLA DRa chain [Ulrich, et *al.* (1995)], while SEA C-terminal domain residues His187, His225 and Asp227 form a zinc-coordination complex, likely with His-81 from the β chain of an adjoining HLA DR molecule. However, our results have shown that binding of superantigen to the HLA DRβ subunit does not directly stimulate T cells [Ulrich et al. (1995) Nature, Struct. Biol. 2, 554-560], but increases the potential of the bound SEA to interact with the α chain of another HLA DR, thus increasing the biological potency.

A least-squares superimposition of the unbound molecules of modeled SEA and the crystal structure of SEB, aligned according to their structurally conserved α-helical and ß-strand regions, exhibited a global folding pattern which is very similar. Differences between the two structures are calculated to be located primarily in loops of low sequence homologies, with the largest positional deviations occurring between structurally conserved regions of residues 18-20, 30-32, 173-181, 191-194, and the cysteine-loop region (90-111). Only one of these regions in SEB makes significant contact (residue Y94 [Y=tyrosine] in particular) with the HLA-DR1 molecule [Jardetzky, T.S. et al. (1994) Nature 368, 711-718].

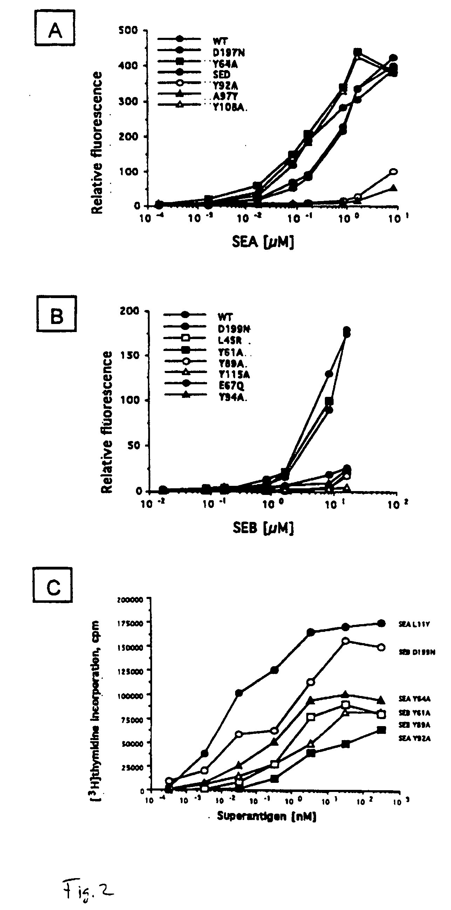

The binding interface between SEB and HLA-DR1 consists principally of two structurally conserved surfaces located in the N-terminal domain: a polar binding pocket derived from three ß-strand elements of the ß-barrel domain and a hydrophobic reverse turn. The binding pocket of SEB contains residues E67 (E=Glutamic acid), Y89 (Y=Tyrosine) and Y115 (Y=tyrosine), and binds K39 (K=Lysine) of the DRα subunit. The amino acid one letter code is defined as the following: A= Alanine (Ala), I= Isoleucine (Ile), L= Leucine (Leu), M= Methionine (Met), F= Phenylalanine (Phe), P= Proline (Pro), W=Tryptophan (Trp), V=Valine (Val), N= Asparagine (Asn), C=Cysteine (Cys), Q= Glutamine (Q), G= Glycine (Gly), S= Serine (Ser), T= Threonine (Thr), Y= Tyrosine (Tyr), R= Arginine (Arg), H=Histidine (His), K= Lysine (Lys), D= Aspartic acid (Asp), and E= Glutamic acid (Glu). For SEA, the binding interface with the DR molecule is modeled to contain a similar binding pocket consisting of residues D70, Y92 and Y108. Mutation of residue Y89 in SEB or Y92 in SEA to alanine (**Fig. 2**) resulted in greater than 100-fold reduction in DR1 binding. The substitution of alanine for Y89 in SEB and Y92 in SEA eliminates the hydrogen bond with K39 and disrupts packing interactions with adjacent protein residues. Modeling of the SEA mutant Y92A predicts an increase in solvent-accessible surface area for Y108 by a factor of two greater than the wild-type structure, allowing the formation of a hydrogen bond to the carboxylate group of D70 and thus disrupting key anchoring and recognition points for HLA-DR1. This effect is expected to be somewhat less in SEB due to the longer side chain at E67. Substitution of SEB Y115 with alanine also resulted in greater than 100-fold reduction of binding. In contrast, the same replacement of Y108 in SEA yielded little to no change in DR1 binding **(****Fig. 2a****),** suggesting the primary importance of SEA residues Y92 and D70 for stabilizing interactions with K39. The K39 side chain of DRα forms a strong ion-pair interaction with the SEB E67 carboxylate group and hydrogen bonds with the hydroxyl groups of Y89 and Y115. Substitution of SEB E67 by glutamine reduced binding affinity by greater than 100-fold (**Fig. 2**), reflecting the replacement of the strong ionic bond with a weaker hydrogen bond. To optimize ion-pair interactions of the analogous SEA site, the shorter carboxylate side chain of D70 is predicted to shift K39 of DRα weakening interactions with SEA Y108. The substitution of alanine for SEA Y108 is thus more easily accommodated than the homologous substitution of SEB Y115, without loss in DR1 binding.

Comparisons of the polar pocket with other bacterial superantigens were then made. SEC1-3 and SPE-A have conserved the critical DR1 binding-interface residues (**Fig. 1**), and share with SEB and SEA secondary structural elements of the DR1-binding surfaces. Asparagine in SED (N70) replaces the acidic side chain present in SEA, SEB, SPE-A and SEC1-3. Accordingly, for SED the salt bridge of the polar pocket is likely to be replaced by a hydrogen bond. Overall, DR1 affinities for SED and SEA appeared to be equivalent (**Fig. 2b**), indicating that other interactions may compensate for the absence in SED of the ion-pair found in the other superantigens. For the case of TSST-1, mutating DRα residues K39 to serine or M36 to isoleucine has been shown to greatly reduce binding [Panina-Bordignon et al. (1992) J. Exp. Med. 176: 1779-1784]. Although primarily hydrophobic, the critical TSST-1 structural elements are conserved with the SEA and SEB polar binding pocket. SEB residues Y89 and Y115 are homologous to T69 and I85 in TSST-1, respectively, and SEB E67 is replaced by I46. These TSST-1 residues are positioned in a conserved ß-barrel domain found in both SEB and SEA. However, the TSST-1 site lacks polarity equivalent to SEB/SEA, and hydrogen bonding with the hydroxyl of TSST-1 residue T69 would require that DRα K39 extend 5 Å into the pocket. TSST-1 binding utilizes an alternative strategy [Kim et al. (1994) Science 266:1870-1874] consisting of hydrophobic contacts centered around residue I46, and potential ionic or hydrogen bonds bridging DRa residues E71 and K67 to R34 and D27, respectively, of TSST-1.

The hydrophobic region of the binding interface between SEB and the HLA-DR1 molecule consists of SEB residues 44-47, located in a large reverse turn connecting ß-strands 1 and 2 of SEB. These residues appear to make strong electrostatic interactions with DRα through their backbone atoms. The mutation of L45 to an arginine reduced overall HLA-DR1 binding greater than 100-fold (**Fig. 2b**), attributable to the less energetically favorable insertion of a highly charged residue into a hydrophobic depression on the DR1 molecule. The modeled DR1-SEA complex presents similar interactions with the SEA backbone atoms, with the exception of a glutamine (Q49) replacing SEB Y46. Mutation of L48 to glycine in SEA (homologous to L45 of SEB) has been reported to decrease T-cell responses. SEB L45 and the comparable L30 of TSST-1 are the most extensively buried residues in the DR1 interface. The leucine is conserved among the bacterial superantigens (**Fig. 3**) and may provide the necessary hydrophobic structural element for surface complementarity with DR1, consistent with the mutagenesis data for SEB and SEA.

The inventor has performed similar structure and function studies with TSST-1, SEC1 and SPE-A.

In determining the overall affinity of the superantigen for DR1, a contributory role is played by structural variations around the common binding motifs. A short, variable structured, disulfide-bonded loop is found in SEA and a homologous longer loop in SEB. The SEB residue Y94, contained within this loop, forms hydrophobic interactions with L60 and A61 of the DRα subunit. Replacement of Y94 with alanine partially inhibits DR1 binding (**Fig. 2a****,b**). An alanine is found in SEA (A97) and SEE at the position equivalent to SEB Y94, and mutating this residue in SEA to tyrosine results in disrupted instead of stabilized interactions with DR1 (**Fig. 2a****).** Although the disulfide loops differ in structure between SEA and SEB, A97 apparently contributes to the DRα binding interface in a manner similar to Y94 of SEB. Because TSST-1 lacks a disulfide loop, similar contacts with DRα are replaced by interactions with β-strands of TSST-1. In a like manner, the absence of a salt bridge between the residues K39 of DRα and N65 of SED is apparently compensated for by stabilizing interactions occurring outside of the otherwise conserved dominant binding surfaces (**Fig. 2a****).**

The amino acid residues in contact with TCR are located in regions of high sequence variability, presenting a unique surface for interaction with the TCR. Residues implicated in TCR interactions by mutagenesis of SEA and SEB reside in variable loop regions, while TSST-1 mutants that affect TCR binding are mainly located in an α helix [Acharya, R.K. et al. (1994) Nature 367, 94-97; Kim, J. et al. (1994) Science 266, 1870-1874]. Specifically, mutations that diminish T-cell receptor recognition of SEB include residues N23, Y61, and the homologous SEA N25 or Y64 **(****Fig. 2c****).** SEA residues S206 and N207 also control T-cell responses [Hudson, et al. (1992) J. Exp. Med. 177: 175-184]. Mutants of the polar binding pocket, SEA Y92A and SEB Y89A, equivalently reduced T-cell responses (**Fig. 2c**), reflecting the observed decreases in DR1-binding (**Fig. 2a****, b**). While supporting reduced T-cell responses, mutants SEA Y64A and SEB Y61A retained normal affinities for DR1 (**Fig. 2a****-c**).

In view of the detailed description of the present invention and the results of molecular modelling and structural studies of staphylococcal and streptococcal superantigen toxins discussed above, any amino acid sequence derived from a superantigen toxin can be altered. Sequences of several superantigen toxins are already known and available to the public in sequence databases such as GenBank, for example. The superantigen toxin sequence is preferably altered at the hydrophobic loop or polar binding pocket depending on the superantigen. Alternatively, residues adjacent to the hydrophobic loop or polar binding pocket that contact HLA-DR or residues at sites that can indirectly alter the structure of the hydrophobic loop or polar pocket can be altered. The number of residues which can be altered can vary, preferably the number can be 1-2, more preferably 2-3, and most preferably 3-4, or more with the limitation being the ability to analyze by computational methods the consequences of introducing such mutations. The residues which can be altered can be within 5 amino acid residues of the central Leucine of the hydrophobic loop (such as L45 of SEB), or within 5 residues of one of the amino acid residues of the polar binding pocket that can contact HLA-DR, (such as E67, Y89, or Y115 of SEB), more preferably, within 3 amino acid residues of the central Leucine of the hydrophobic loop (such as L45 of SEB), or within 3 residues of one of the amino acid residues of the polar pocket that can contact HLA-DR, (such as E67, Y89, or Y115 of SEB), and most preferably, the central Leucine of the hydrophobic loop (such as L45 of SEB), or one of the amino acid residues of the polar binding pocket that can contact HLA-DR, (such as E67, Y89, or Y115 of SEB). The residues can be changed or substituted to alanine for minimal disruption of protein structure, more preferably to a residue of opposite chemical characterisitcs, such as hydrophobic to hydrophilic, acidic to neutral amide, most preferably by introduction of a residue with a large hydrated side chain such as Arginine or Lysine. In addition, side chains of certain nonconserved receptor-binding surfaces, can also be altered when designing superantigen toxins with low binding affinities. These residues can include Y94 of SEB and structurally equivalent residues of other superantigens, such as A97 of SEA, or any side chain within 5 residues from these positions or any side chain in discontinuous positions (discontinuous positions are defined as amino acid residues that fold together to form part of a discrete three-dimensional structural unit but are not present on the same secondary structural unit e.g. α helix or β-strand) such as disulfide-bonded side chains, that involve, directly or indirectly, the nonconserved receptor contact surfaces outside of the polar binding pocket or hydrophobic loop. Further, amino acid residues involved with protein folding or packing can be altered when designing superantigen toxins with low binding affinities [Sundstrom et al. (1996) EMBO J. 15, 6832-6840; Sundstrom et al. (1996) J. Biol. Chem. 271, 32212-32216; Acharya et al. (1994) Nature 367, 94-97; Prasad et al. (1993) Biochem. 32, 13761-13766; Swaminathan et al. (1992) Nature 359, 801-806]. Furthermore, especially for superantigens with higher affinities for T-cell antigen receptors, side chains of amino acids within 5 residues of the position represented by N23 (conserved residue in most superantigens), N60 (conserved Asn or Trp in most superantigens) Y91 (semiconserved hydrophobic residues Trp, Ile, Val, His in most superantigens) and D210 of SEB (conserved Asp in most superantigens) can be altered when designing superantigen toxins with low binding affinities. These residues are likely to form part of the integral molecular surfaces that are in contact with T-cell antigen receptors. Because the T-cell receptor contact areas of superantigen toxins are essential for causing specific activation or inactivation of T-cell subsets, altering residues that are unique to each superantigen but that are located within 5 residues of the positions represented by N23, N60 and Y91 can produce superantigens that affect a smaller number (e.g. 1-3) of subsets. Such altered superantigen toxins can be useful as therapeutic agents.

In another embodiment, the present invention relates to a DNA or cDNA segment which encodes an SEA superantigen toxin, the sequence of which has been altered as described above to produce a toxin protein with altered binding ability to MHC Class II and/or T-cell receptors. There is also disclosed DNA or cDNA segment which encodes a superantigen toxin SEB, SEC-1, SPEa, and TSST-1 to name a few. For SEA, the following three mutations were introduced into the toxin molecule: Tyrosine at amino acid position 92 changed to alanine; Aspartic acid at amino acid position 70 changed to arginine; Leucine at amino acid position 48 changed to arginine. The reduction in binding to HLA DR is additive per mutation, though one or two mutations can produce a vaccine and a combination of all three mutations in one molecule produces a better vaccine. Other substitutions can also result in reduced binding.

The B899445 vaccine consists of the following three mutations simultaneously introduced into the toxin molecule: tyrosine at amino acid position 89 changed to alanine; tyrosine at amino acid position 94 changed to alanine; leucine at amino acid position 45 changed to arginine. The altered superantigen toxins can be expressed either as a full-length propolypeptide or as a polypeptide in which the leader peptide has been deleted. The full-length expressed product (SEA vaccine, A489270P; SEB vaccine B899445P, B2360210P) is secreted into the periplasmic space of *E. coli* host cells, and the leader peptide is recognized and cleaved by a native bacterial enzymatic mechanism. The altered superantigen toxins in which the leader peptide has been deleted (A489270C, B899445C), the first residue of the mature protein is encoded by the transcriptional start site and codon for methionine (ATG), and the protein is expressed as a nonsecreted product within the host *E*. *coli* cell. For the TSST-1 vaccine TST30, the leucine at position 30 was changed to arginine. For the SEC1 vaccine, SEC45, the leucine at position 45 was changed to arginine. For the SPE-A vaccine, SPEA42, the leucine at position 42 was changed to arginine.

In another embodiment, the present invention relates to a recombinant DNA molecule that includes a vector and a DNA sequence as described in the embodiment above. The vector can take the form of a plasmid such as any broad host range expression vector for example pUC18/19, pSE380, pHIL, pET21/24 and others known in the art. The DNA sequence is preferably functionally linked to a promoter such that the gene is expressed when present in an expression system and an altered superantigen toxin is produced. The expression system can be an *in vitro* expression system or host cells such as prokaryotic cells, or *in vivo* such as DNA vaccines.

In a further embodiment, the present invention relates to host cells stably or transiently transformed or transfected with the above-described recombinant DNA construct embodiments. The host can be any eukaryotic or prokaryotic cell including but not limited in *E. coli* DH5α or BL21. The vector containing the altered superantigen toxin gene is expressed in the host cell and the product of the altered toxin gene, whether a secreted mature protein or a cytoplasmic product, can be used as a vaccine or as a reagent in diagnostic assays or detection methods, or for therapeutic purposes. Please see e.g., Maniatis, Fitsch and Sambrook, Molecular Cloning; A Laboratory Manual (1982) or DNA Cloning, Volumes I and II (D. N. Glover ed. 1985) for general cloning methods. The DNA sequence can be present in the vector operably linked to a highly purified IgG molecule, an adjuvant, a carrier, or an agent for aid in purification of altered toxin. The transformed or transfected host cells can be used as a source of DNA sequences described above. When the recombinant molecule takes the form of an expression system, the transformed or transfected cells can be used as a source of the altered toxin described above.

A recombinant or derived altered superantigen toxin is not necessarily translated from a designated nucleic acid sequence; it may be generated in any manner, including for example, chemical synthesis, or expression of a recombinant expression system. In addition the altered toxin can be fused to other proteins or polypeptides for directing transport for example into the periplasm or for secretion from the cell. This includes fusion of the recombinant or derived altered superantigen to other vaccines or sequences designed to aid in purification, such as His-tagged, epitope-tagged or antibody Fc-fusions.

In a further embodiment, the present invention relates to a method of producing altered superantigen toxin which includes culturing the above-described host cells, under conditions such that the DNA fragment is expressed and a superantigen toxin protein is produced. The superantigen toxin can then be isolated and purified using methodology well known in the art such as immunoaffinity chromatography or preparative isoelectric focusing. However, the method of purification is not critical to the performance of the vaccine. The altered superantigen toxin can be used as a vaccine for immunity against infection with bacterial superantigen toxins or as a diagnostic tool for detection of superantigen toxin-associated disease or bacterial infection. The transformed host cells can be used to analyze the effectiveness of drugs and agents which affect the binding of superantigens to MHC class II or T-cell antigen receptors. Chemically derived agents, host proteins or other proteins which result in the down-regulation or alteration of expression of superantigen toxins or affect the binding affinity of superantigen toxins to their receptors can be detected and analyzed. A method for testing the effectiveness of a drug or agent capable of altering the binding of superantigen toxins to their receptors can be for example computer-aided rational design or combinatorial library screening, such as phage display technology.

There are disclosed antibodies specific for the above-described altered superantigen toxins. For instance, an antibody can be raised against the complete toxin or against a portion thereof. Persons with ordinary skill in the art using standard methodology can raise monoclonal and polyclonal antibodies to the altered superantigens of the present invention, or a unique portion of the altered superantigen. Materials and methods for producing antibodies are well known in the art (see for example Goding, in, Monoclonal Antibodies: Principles and Practice, Chapter 4, 1986). The antibodies can be used in diagnostic assays for detection of superantigen toxin-associated infection. Neutralizing antibodies can be used in a therapeutic composition for the treatment of amelioration of anergy and/or for the treatment of a superantigen toxin-associated infection.

In a further embodiment, the present invention relates to a method for detecting the presence of superantigen-associated bacterial infections in a sample. Using standard methodology well known in the art, a diagnostic assay can be constructed by coating on a surface (i.e. a solid support) for example, a microtitration plate or a membrane (e.g. nitrocellulose membrane), all or a unique portion of the altered superantigen described above, and contacting it with the serum of a person suspected of having a superantigen-associated bacterial infection. The presence of a resulting complex formed between the altered superantigen toxin and antibodies specific therefor in the serum can be detected by any of the known methods common in the art, such as fluorescent antibody spectroscopy or colorimetry. This method of detection can be used, for example, for the diagnosis of superantigen-associated bacterial infections.

In yet another embodiment, the present invention relates to a method for detecting the presence of superantigen toxin in a sample. Using standard methodology well known in the art, a diagnostic assay can be constructed by coating on a surface (i.e. a solid support) for example, a microtitration plate or a membrane (e.g. nitrocellulose membrane), antibodies specific for altered superantigen toxin, and contacting it with serum or tissue sample of a person suspected of having superantigen-associated bacterial infection. The presence of a resulting complex formed between toxin in the serum and antibodies specific therefor can be detected by any of the known methods common in the art, such as fluorescent antibody spectroscopy or colorimetry. This method of detection can be used, for example, for the diagnosis of superantigen-associated bacterial infection or disease such as food poisoning and toxic-shock syndrome or the detection of superantigen toxin in food and drink.

In another embodiment, the present invention relates to a diagnostic kit which contains altered superantigen toxin from a specific bacteria or several different superantigen toxins from bacteria and ancillary reagents that are well known in the art and that are suitable for use in detecting the presence of antibodies to superantigen toxin-associated bacteria in serum or a tissue sample. Tissue samples contemplated can be avian, fish, or mammal including monkey and human.

In yet another embodiment, the present invention relates to a vaccine for protection against superantigen toxin-associated bacterial infections. The vaccine can comprise one or a mixture of individual altered superantigen toxins, or a portion thereof. When a mixture of two or more different altered superantigen toxin from different bacteria is used, the vaccine is referred to as a multivalent bacterial superantigen vaccine. The vaccine is designed to protect against the pathologies resulting from exposure to one or several related staphylococcal and streptococcal toxins. In addition, the protein or polypeptide can be fused or absorbed to other proteins or polypeptides which increase its antigenicity, thereby producing higher titers of neutralizing antibody when used as a vaccine. Examples of such proteins or polypeptides include any adjuvants or carriers safe for human use, such as aluminum hydroxide.

The staphylococcal enterotoxin (SE) serotypes SEA, SED, and SEE are closely related by amino acid sequence, while SEB, SEC1, SEC2, SEC3, and the streptococcal pyrogenic exotoxins B share key amino acid residues with the other toxins, but exhibit only weak sequence homology overall. However, there are considerable similarities in the known three-dimensional structures of SEA, SEB, SEC1, SEC3, and TSST-1. Because of this structural similarity, it is likely that polyclonal antibodies obtained from mice immunized with each SE or TSST-1 exhibit a low to high degree of cross-reaction. In the mouse, these antibody cross-reactions are sufficient to neutralize the toxicity of most other SE/TSST-1, depending upon the challenge dose. For example, immunization with a mixture of SEA, SEB, TSST-1 and SPEa was sufficient to provide antibody protection from a challenge with any of the component toxins, singly or in combination.

The likelihood of substantial
antigen-cross-reactivity suggests that it may be possible to obtain immune protection for other (or perhaps all) staphylococcal superantigens by use of a minimal mixed composition of vaccines. For the case of staphylococcal superantigens, a combination of the component vaccines from SEA, SEB, SEC-1 and TSST-1 should be sufficient to provide immune protection against SEA, SEB, SEC1-3, and TSST-1. The addition of SPEa component to the trivalent mixture will allow for sufficient protection against the streptococcal toxins SPEa and SPEc. Therefore, a multivalent vaccine consisting of the altered superantigen toxins from SEA, SEB, SEC-1, TSST-1, and SPEa as described above, is predicted to provide protective immunity against the majority of bacterial superantigen toxins.

The vaccine can be prepared by inducing expression of a recombinant expression vector comprising the gene for the altered toxin described above. The purified solution is prepared for administration to mammals by methods known in the art, which can include filtering to sterilize the solution, diluting the solution, adding an adjuvant and stabilizing the solution. The vaccine can be lyophilized to produce a vaccine against superantigen toxin-associated bacteria in a dried form for ease in transportation and storage. Further, the vaccine may be prepared in the form of a mixed vaccine which contains the altered superantigen toxin(s) described above and at least one other antigen as long as the added antigen does not interfere with the effectiveness of the vaccine and the side effects and adverse reactions, if any, are not increased additively or synergistically. Furthermore, the vaccine may be administered by a bacterial delivery system and displayed by a recombinant host cell such as *Salmonella* spp, *Shigella* spp, *Streptococcus* spp. Methods for introducing recombinant vectors into host cells and introducing host cells as a DNA delivery system are known in the art [Harokopakis et al. (1997) Infect. Immun. 65, 1445-1454; Anderson et al. (1996) Vaccine 14, 1384-1390; Medaglini et al. (1995) Proc. Natl. Acad. Sci. U.S.A. 92, 6868-6872].

The vaccine may be stored in a sealed vial, ampule or the like. The present vaccine can generally be administered in the form of a liquid or suspension. In the case where the vaccine is in a dried form, the vaccine is dissolved or suspended in sterilized distilled water before administration. Generally, the vaccine may be administered orally, subcutaneously, intradermally or intramuscularly but preferably intranasally in a dose effective for the production of neutralizing antibody and protection from infection or disease.

There is disclosed a method of reducing superantigen-associated bacterial infection symptoms in a patient by administering to said patient an effective amount of anti-altered superantigen toxin antibodies, as described above. When providing a patient with anti-superantigen toxin antibodies, or agents capable of inhibiting superantigen function to a recipient patient, the dosage of administered agent will vary depending upon such factors as the patient's age, weight, height, sex, general medical condition, previous medical history, etc. In general, it is desirable to provide the recipient with a dosage of the above compounds which is in the range of from about 1pg/kg to 10 mg/kg (body weight of patient), although a lower or higher dosage may be administered.

There is disclosed a therapeutic method for the treatment of diseases that may not be associated directly with superantigen toxins but which result in specific nonresponsiveness of T-cell subsets or detection of abnormally low level of subsets in peripheral blood, said method comprising the administration of altered superantigen toxins, *in vivo* or *ex vivo,* such that T-cell subsets are expanded or stimulated. Diseases which cause anergy or nonresponsiveness of T-cells include, but are not limited to, infectious diseases and cancers. The desired clinical outcome such as an increase in detectable T cell subsets or in stimulation *ex vivo* of T-cells through their antigen receptors, such as by antigen or anti-CD3 antibody can be measured by standard clinical immunology laboratory assays.

There is disclosed a therapeutic method for the treatment of diseases associated with expanded or over-stimulated T-cell subsets, such as autoimmunity for example, said method comprising administration *in vivo* or *ex vivo,* of superantigen toxin altered in such a manner that only limited (1-3) T-cell subsets are stimulated but that MHC class II binding affinity still remains, such that anergy or inactivation of T-cells is produced. The desired clinical outcome can be measured as a reduction of circulating blood T-cells of the targeted subset(s) or diminished antigen or other antigen receptor-mediated-stimulatory responses by assays known in the art.

Described below are examples of the present invention which are provided only for illustrative purposes. In light of the present disclosure, numerous embodiments within the scopy of the claims will be apparent to those of ordinary skill in the art.

The following Materials and Methods were used in the Examples that follow.

### Structural comparisons

Primary protein structure data are available for several bacterial superantigens, including SEA, SED, SEB, SEC1-3, TSST-1. Superantigens for which structures were unavailable were modeled using comparative techniques (HOMOLOGY program; Biosym Technologies, Inc., San Diego, CA). Before x-ray crystallography data was available, SEA was modeled by using this method, and the model was in very close agreement with the experimentally determined structure. As an example, the amino acid sequence for SEA was aligned with the known structure of free and HLA-DR1 bound SEB, and the SEA molecule was built for both free and DR1-bound proteins. Loop segments of SEA were generated by a *de novo* method. Refinement of the modeled structures was carried out by means of molecular-dynamics simulations (DISCOVER, Biosym). The constructed free SEA molecule was immersed in a 5-Å layer of solvent water and the a-carbon atoms lying in the structurally conserved regions were tethered to their initial positions during the simulations. For the bound SEA molecule, simulations were carried out by constructing an active-site region composed of part of the SEA molecule and the DR1 molecule inside a 10-Å interface boundary, as derived from the crystal structure of the DR1-SEB complex. Amino acid residues lying in the outer boundary were rigidly restrained at their initial positions. The active-site region was immersed in a 5-Å layer of water. Protein interactions were modeled by employing the consistent valence force field with a non-bonded cutoff distance of 11.0 A. Simulations were initiated with 100 cycles of minimization using a steepest descent algorithm followed by 100-ps relaxation (using a 1.0 fs timestep). Structural comparisons between SEB, SEC1, and TSST-1 were performed by using the crystal structures (Brookhaven data holdings) aligned according to common secondary structural elements and/or by sequence and structural homology modeling.

### Site-specific mutagenesis

Site-specific mutagenesis was performed according to the method developed by Kunkel, using gene templates isolated from Staphylococcus aureus strains expressing SEA (FDA196E, a clinical isolate, Fraser, J.D. (1994) Nature 368: 711-718), SEB (14458, clinical isolate), SEC1 (Toxin Technologies, Sarasota, FL), TSST-1 (pRN6550 cloned product, a clinical isolate, Kreiswirth, B. N. et al. (1987) Mol. Gen. Genet. 208, 84-87), and SPEa (Toxin Technologies), respectively. Modified T7 polymerase (Sequenase, U.S. Biochemical Corp., Cleveland, OH) was used to synthesize second-strand DNA from synthetic oligonucleotides harboring the altered codon and single-stranded, uracil-enriched M13 templates. Mutagenized DNA was selected by transforming E. *coli* strain JM101. Alternatively, double stranded DNA was used as template for mutagenesis. Mutagenized sequences were confirmed by DNA sequencing (Sanger et al., 1977, Proc. Natl. Acad. Sci. USA 74: 5463-5467; Sambrook *et al.,* 1989) using synthetic primers derived from known sequences, or universal primers. The complete coding sequences were inserted into expression plasmids such as pUC19, pSE380 or pET21 for production in *E. coli* hosts.

### Protein purifications

The appropriate *E. coli* hosts were transformed with plasmids harboring the mutant toxin genes. In general, the bacteria were grown to an A600 0.5-0.6 in Terrific Broth (Difco Laboratories, Detroit, MI) containing 50 µg/mL ampicillin or kanamycin. Recombinant proteins were induced with isopropyl-ß-D-thio-galactopyranoside (Life Technologies, Gaithersburg, MD) and recovered as cytoplasmic or bacterial periplasmic secretion products. Bacteria were collected by centrifugation, washed with 30 mM NaCl, 10 mM TRIS (pH 7.6), and pelleted by centrifugation and either lysed or osmotically shocked for collection of secreted proteins. Preparations were isolated by CM Sepharose ion-exchange chromatography, rabbit antibody (Toxin Technologies, Sarasota, FL) affinity columns, ion exchange HPLC or similar methods. In some cases partially purified superantigen was further purified by preparative isoelectric focusing (MinipHor; Rainin Instrument Company, Inc., Woburn, MA.). The MinipHor was loaded with the SEA-enriched fraction from CM Sepharose chromatography in a solution containing 10% (v/v) glycerol and 1% (v/v) pH 6-8 ampholytes (Protein Technologies, Inc., Tucson, AZ). The protein preparations were allowed to focus until equilibrium was reached (approximately 4 hr, 4°C). Twenty focused fractions were collected and aliquots of each were analyzed by SDS-polyacrylamide gel electrophoresis (SDS-PAGE) and immunoblotting. The SEA-containing fractions were pooled, and refocused for an additional 4 h. The fractions containing purified SEA were pooled and dialyzed first against 1 M NaCl (48 h, 4°C) to remove ampholytes, and then against PBS (12 h, 4°C). Legitimate amino-terminal residues were confirmed by protein sequencing. Precise measurements of protein concentrations were performed by immunoassay using rabbit antibody affinity-purified with the wild-type superantigens and by the bicinchoninic acid method (Pierce, Rockford, IL) using wild-type protein as standards. All protein preparations were >99% pure, as judged by SDS-PAGE and Western immunoblots. In some cases, as when used for lymphocyte assays, bacterial pyrogens were removed by passing the protein preparations over Polymyxin B affinity columns.

### Binding of superantigens to HLA-DR1

The DR1 homozygous, human B-lymphoblastoid cell line LG2 or L cells transfected with plasmids encoding HLA-DR1αβ were used in the binding experiments. Cells were incubated 40 min (37°C) with wild-type or mutant superantigen in Hanks balanced salt solution (HBSS) containing 0.5% bovine serum albumin. The cells were washed with HBSS and then incubated with 5 µg of specific rabbit antibody (Toxin Technology, Sarasota, FL) for 1 h on ice. Unbound antibody was removed, and the cells were incubated with FITC-labelled goat anti-rabbit IgG (Organon Teknika Corp., Durham, N.C.) on ice for 30 min. The cells were washed and analyzed by flow cytometry (FACScan; Becton Dikinson & Co., Mountain View, CA). Controls consisted of cells incubated with affinity purified anti-toxin and the FITC labelled antibody without prior addition of superantigen.

### Lymphocyte proliferation

Human peripheral blood mononuclear cells were purified by Ficoll-hypaque (Sigma, St. Louis, MO) buoyant density gradient centrifugation. Genes encoding the human MHC class II molecules DR1αβ (DRA and DRB1*0101 cDNA [Bavari and Ulrich (1995) Infect. Immun. 63, 423-429] were cloned into the eukaryotic expression vector pRC/RSV (Invitrogen, Carlsbad, CA), and mouse L cells were stably transfected. The transfectants were selected by fluorescence-activated cell sorting (EPICS C, Coulter Corp., Hialeah, FL) using rabbit anti-DRαβ antisera and FITC-goat anti-rabbit IgG, to produce cells that expressed a high level of DRαβ21. 1 x 10⁵ cells/well of a 96-well plate were irradiated (15,000 Rad), and wild-type or mutant SE, was added. After a brief incubation period (45 min, 37°C), unbound SE was rinsed from the culture plates using warm media. The cells were cultured in RPMI-1640 (USAMRIID) with 5% FBS for 72 h, and pulsed-labelled for 12 h with 1µCi [³H]-thymidine (Amersham, Arlington Heights, IL). Cells were harvested onto glass fiber filters, and [³H]-thymidine incorporation into the cellular DNA was measured by a liquid scintillation counter (BetaPlate, Wallac Inc., Gaithersburg, MD). Splenic mononuclear cells or human peripheral blood mononuclear cells were obtained by buoyant density centrifugation (Histopaque; Sigma Chemical Comp.) and washed three times. The cells were resuspended in medium containing 5% fetal bovine serum (FBS), and 100 µl (4 x 10⁵ cells) of the cell suspension was added to triplicate wells of 96-well flat bottom plates. The mononuclear cells were cultured (37°C, 5% CO₂) with WT or mutant SEA. After 3 days the cultures were pulsed (12h) with 1 µCi/well of [³H]thymidine (Amersham, Arlington Heights, IL) and incorporated radioactivity was measured by liquid scintillation.

### Gel electrophoresis and immunoblotting analysis.

The protein preparations were analyzed by SDS-PAGE (12%) and stained with Coomassie Brilliant Blue R-250 (Sigma Chemical Comp. St Louis, MO) in methanol (10% v/v) acetic acid (10% v/v). The proteins separated by SDS-PAGE (not stained) were transferred to nitrocellulose membranes (Bio-Rad Lab. Inc., Melville, NY) by electroblotting, and the membranes were then blocked (12 h, 4°C) with 0.2% casein in a buffer consisting of 50 mM sodium phosphate, 140 mM sodium chloride, pH 7.4 (PBS). The membrane was then incubated (1 h, 37°C, shaking) with 2 µg/mL of affinity-purified anti-toxin antibody (Toxin Technology, Sarasota, FL) in PBS with 0.02% casein. After the membranes were thoroughly washed, peroxidase-conjugated goat anti-rabbit IgG (Cappel/Organon Teknika Corp., West Chester, PA) was added (1:5,000) and the membranes were incubated for 1 h (37°C) with shaking. The unbound antibody was removed by washing with PBS and bound antibody was visualized by using a Bio-Rad peroxidase development kit (Biorad, Hercules, CA). For quantitation, dilutions of wild-type preparations were immobilized on nitrocellulose membranes by using a Slot-Blot apparatus (Bio-Rad). The membrane was removed from the Slot-Blot apparatus and unreacted sites were blocked (12h, 4°C) with 0.2% casein in PBS. After washing once with the PBS, the membrane was incubated (1h, 37°C) with 2 µg/mL rabbit affinity purified anti-toxin antibody (Toxin Technology) in PBS that contained 0.02% casein. After four washes, the bound rabbit antibody was reacted with goat anti-rabbit IgG conjugated with horseradish peroxidase (1 h, 37°C) and the blots were developed using enhanced chemiluminescence (ECL; Amersham Life Sciences, Arlington Heights, IL) or similar methods. The amount of mutant protein was measured by densitometry (NIH Image 1.57 software, National Institutes of Health, Bethesda, MD) of exposed X-ray film. Standard curves were prepared by plotting the mean of duplicate densitometric readings for each dilution of toxin standard. The resulting values were fitted to a straight line by linear regression. Concentrations of proteins were determined by comparing mean values of various dilutions of the mutant to the standard curve.

### Biological activities and Immunizations.

Male C57BL/6 mice, 10 to 12-weeks old, were obtained from Harlan Sprague-Dawley, Inc. (Frederick Cancer Research and Development Center, Frederick, MD). The lethal effect of WT or mutant SEA was evaluated as described in Stiles et al. (1993) Infect. Immun. 61, 5333-5338. For immunizations, mice were given by interperitoneal (ip) injections either 2 or 10 µg of WT or mutant toxin in 100 µl of adjuvant (RIBI, Immunochem Research, Inc. Hamilton, MT or alum), or adjuvant only, and boosted (ip) at 2 and 4 weeks. Serum was collected from tail veins one week after the last immunization. Mice were challenged 2 weeks after the last injection with toxin and lipopolysaccharide (LPS, 150 µg) from *E. coli* 055:B5 serotype (Difco Laboratories, Detroit, MI). Challenge controls were adjuvant-immunized or non-immunized mice injected with both agents (100% lethality) or with either wild type toxin or LPS. No lethality was produced by these negative controls. Monkeys were immunized with the antigen in the right leg, caudal thigh muscles. Each received three intramuscular immunizations with a superantigen vaccine plus adjuvant. Control monkeys received 0.5 ml total volume of adjuvant (Alhydrogel, Michigan Department of Public Health) and sterile PBS using the same techniques and equipment as the immunized monkeys. Immunizations were administered 28±2 days apart and consisted of 20 µg of the vaccine in adjuvant in a total volume of 0.5 ml. Immunizations were administered on day 0, 28±2, and 56±2 using a 23-27 ga 1/2-5/8" needle attached to a 1 ml tuberculin syringe into the caudal thigh.

### Antibody assay.

Microtiter plates were coated with 1 µg/well of WT toxin in 100 µl of PBS (37°C, 2 h). After antigen coating, the wells were blocked with 250 µl of casein 0.2% in PBS for 4 h at 37°C and then washed four times with PBS containing 0.2% Tween 20. Immune or nonimmune sera were diluted in PBS containing 0.02% casein and 100 µl of each dilution was added to duplicate wells. After each well was washed four times, bound antibody was detected with horse radish peroxidase (Sigma Chemical Comp., St. Louis, MO) labelled goat anti-species specific IgG (37°C, 1 h), using O-phenylenediamine as the chromogen. Mean of duplicates OD (absorbance at 490 nm) of each treatment group was obtained and these data were compared on the basis of the inverse of the highest serum dilution that produced an OD reading four times above the negative control wells. For negative controls, antigen or serum was omitted from the wells.

### Superantigen binding and TCR subset analysis.

Cells from the mouse B-lymphoma line A20 (ATCC, Rockville, MD) (2-4 x 10⁵ cells) were incubated (40 min at 37°C) with WT or mutant toxin in Hanks balanced salt solution containing 0.5% bovine serum albumin (HBSS, USAMRIID). The cells were washed with HBSS and incubated with 5 µg of affinity-purified anti-toxin antibody in HBSS (4°C, 45 min). Unbound antibody was removed and the bound antibody was detected with fluorescein isothiocyanate (FITC)-labelled, goat anti-rabbit IgG (Organon Teknika Corp., Durham, NC). Unbound antibody was removed and the cells were analyzed by with a FACSort flow cytometer (Becton Dikinson & Co. , Mountain View, CA).

For TCR subset analysis, splenic mononuclear cells were obtained from mice immunized with WT or mutant toxin. The mononuclear cells were incubated (37°C) with WT toxin (100 ng/mL) for 5 days and then cultured in 85% RPMI-1640, 10% interleukin-2 supplement (Advanced Biotechnologies Inc., Columbia, MD) with 5% FBS for an additional 5 days. The T cells were washed twice and stained with anti-TCR (Biosource, Camarillo, CA) or anti-Vß specific TCR (Biosource, Camarillo, CA) (45 min, 4°C). All cells analyzed were positive for T cell marker CD3+ and expressed the CD25 activation marker (data not shown). Controls were incubated with an isotype matched antibody of irrelevant specificity. Unreacted antibody was removed, and the cells were incubated with an FITC-labelled, anti-mouse IgG (Organon Teknika Corp, Durham, NC) on ice for 30 min. The cells were washed and analyzed by flow cytometry (FACSort).

### LPS potentiation of SE toxicity in mice.

C57BL/6 or BALB/c mice weighing 18-20 g (Harlan Sprague Dawley, Inc., Frederick Cancer Research and Development Center, Frederick, MD) were each injected intraperitoneally (i.p.) with 200 µl of PBS containing varying amounts of SEA, SEB, or SEC1, TSST-1, or SPEa followed 4 h later with 75 or 150 µg of LPS (200 µl/i.p.). Controls were each injected with either SE (30 mg) or LPS (150 mg). Animals were observed for 72 h after the LPS injection. Calculations of LD50 were done by Probit analysis using 95% fiducial limits (SAS Institute Inc., Cary, NC).

The biological effects of SEA and SEB were also tested in transgenic C57BL/6 mice (GenPharm International, Mountain View, CA) deficient in MHC class I or II expression [Stiles et al. (1993) Infect. Immun. 61, 5333-5338], as described above, using a single dose of toxin (30 µg/mouse). Genetic homozygozity was confirmed by Southern analysis of parental tail DNA, using β2 microglobulin and MHC class IIβ DNA probes.

### Detection of cytokines in serum.

Mice (n=18 per group) were injected with toxin (10 µg), LPS (150 µg), or toxin plus LPS. Sera were collected and pooled from three mice per group at each time point (2, 4, 6, 8, 10, 22 h) after LPS injection. Sera were collected at various time points following toxin injection (-4 h, or 4h before LPS injection, for data tabulation). Collection of LPS control sera began at the time of injection (0 h).

Serum levels of TNFα and IL-α were detected by an enzyme linked immunosorbent assay (ELISA). TNFα was first captured by a monoclonal antibody against mouse TNFα (GIBCO-BRL, Grand Island, NY) and then incubated with rabbit anti-mouse TNFα antibody (Genzyme, Boston, MA). The ELISA plate was washed and peroxidase conjugate of anti-rabbit antibody (Boehringer Mannheim, Indianapolis, IN) added to the wells. After washing the plate and adding substrate (Kirkegaard and Perry, Gaithersburg, MD), TNFα concentrations were measured using the mean A450 reading of duplicate samples and a standard curve generated from recombinant mouse TNFα (GIBCO-BRL). Serum levels of IL-1α were determined from the mean reading of duplicate samples with an ELISA kit that specifically detects murine IL-1α (Genzyme, Boston, MA). The standard error of the mean (SEM) for TNF α and IL-1α readings was +/- 5%.

Quantitation of IL-6 and IFNg were measured by bioassays [See et al. (1990) Infect. Immun. 58: 2392-2396]. An IL-6 dependent cell line, 7TD1 (kindly provided by T. Krakauer), was used in a proliferative assay with serial two-fold dilutions of serum samples assayed in triplicate. Proliferation of 7TD1 cells in a microtiter plate was measured by uptake of [³H]-thymidine (1 µCi/well; Amersham, Arlington Heights, IL) and the activity of IL-6 from serum was compared to a recombinant mouse IL-6 standard (R and D Systems, Minneapolis, MN) as previously described [See et al.(1990) Infect. Immun. 58: 2392-2396]. The SEM of triplicate samples was +/- 10%.

IFNg was measured by the reduction of vesicular stomatitis virus (New Jersey strain) cytopathic effects on L929 cells, as previously described [Torre et al.(1993) J. Infect. Dis. 167, 762-765]. Briefly, serial two-fold dilutions of serum were made in duplicate and added to microtiter wells containing L929 cells (5 x 10⁴/well). After incubating 24 h, virus (5 x 10⁵ PFU/well) was added and the cytopathic effects measured at 48 h by absorbance readings (570 nm) of reduced 3-[4, 5-dimethylthiazol-2-yl]-2,5 diphenyl tetrazolium bromide (Sigma). The activity of each serum sample was determined using recombinant mouse IFNγ as a standard (Biosource, Camarillo, CA). The SEM of duplicate samples was +/- 5%.

### EXAMPLE 1

### Molecular modelling and structural studies of staphylococcal and streptococcal superantigens: bacterial superantigens share common 3-dimensional structure.

Comparison of amino acid sequences (**Fig. 1**) suggested that bacterial superantigens fall into groups consisting of (1) SEA, SED and SEE, (2) SEB, staphylococcal enterotoxins C1-C3 (SEC1-3), the streptococcal pyrogenic exotoxins A (SPE-A) and C (SPE-C), (3) TSST-1 and (4) the exfoliative toxins (ETA, ETB) and streptococcal pyrogenic exotoxin B (SPE-B), which are the most distant from the others in sequence. Although not available to the inventor when the inventions were first conceived and proof of principle was obtained, the x-ray crystallographic structures of several bacterial superantigens are now known. Diverse superantigens, such as SEB and TSST-1, appear to have little sequence in common, yet they exhibit homologous protein folds composed largely of β strands [Prasad, G.S. et al. (1993) Biochemistry 32, 13761-13766; Acharya, R.K. et al. (1994) Nature 367, 94-97; Swaminathan, S. et al. (1992) Nature 359, 801-806] within two distinct domains. Differences between the proteins are located primarily in highly variable regions comprised of several surface loops, such as the disulfide-bonded loop which is absent from TSST-1 and at the amino terminus.

The X-ray crystal structures of SEB and TSST-1 complexed with HLA DR1 are known[Kim, J. et al. (1994) Science 266, 1870-1874 ; Jardetzky, T.S. et al. (1994) Nature 368, 711-718] and this data was useful to fully explain our results concerning attenuation of the superantigens by site-specific mutagenesis. The region of HLA DR1 that contacts SEB consists exclusively of α subunit surfaces. The main regions of SEB involved are two conserved sites: a polar pocket derived from three β strands of the β barrel domain and a highly solvent-exposed hydrophobic reverse turn. The polar binding pocket of SEB contains a glutamate and two tyrosines that accommodate Lys39 of the α subunit of HLA DR1, while the hydrophobic region consists of a leucine and flanking residues that make several contacts with the HLA DRα chain. The HLA DR1 binding sites of both TSST-1 and SEB overlap significantly. The hydrophobic binding contacts of other SAg with the HLA DRα chain have been proposed [Ulrich et al. (1995) Nature, Struct. Biol. 2, 554-560] to be similar to those found in SEB and TSST-1. A motif consisting of a leucine in a reverse turn [Ulrich *et al.* (1995), supra] is conserved among bacterial superantigens and may provide the key determinant (hydrophobic or otherwise) for binding HLA-DR. However, TSST-1 does not have a highly charged residue in the polar pocket that interacts with Lys39 of the HLA DRα chain and uses an alternative conformational binding mode that allows TSST-1 to interact with HLA DR1 β -chain residues and the carboxy-terminal region of the antigenic peptide.

Both SEA and SEE bind to the β subunit of DR by means of a single zinc atom [Fraser, J.D. et al. (1992) Proc. Natl. Acad. Sci. USA 89, 5507-5511]. The amino-terminal domain of SEA interfaces with the HLA DR α chain [Ulrich et *al.* (1995), supra], while SEA C-terminal domain residues His187, His225 and Asp227 form a zinc-coordination complex, likely with His-81 from the β chain of an adjoining HLA DR molecule. However, our results have shown that binding of superantigen to the HLA DR β subunit does not directly stimulate T cells [Ulrich *et al*. (1995), supra] but increases the potential of the bound SEA to interact with the α chain of another HLA DR, thus increasing the biological potency.

### EXAMPLE 2

### Molecular modelling and structural studies of staphylococcal and streptococcal superantigens: A detailed protein structure analysis of SEB and SEA suggested that all bacterial superantigens have a common mechanism for binding MHC class II receptors.

A least-squares superimposition of the unbound molecules of modeled SEA and the crystal structure of SEB, aligned according to their structurally conserved α-helical and ß-strand regions, exhibited a global folding pattern which is very similar. Differences between the two structures are calculated to be located primarily in loops of low sequence homologies, with the largest positional deviations occurring between structurally conserved regions of residues 18-20, 30-32, 173-181, 191-194, and the cysteine-loop region (90-111). Only one of these regions in SEB makes significant contact (residue Y94 in particular) with the HLA-DR1 molecule [Jardetzky, T.S. et al. (1994) Nature 368, 711-718].

The binding interface between SEB and HLA-DR1 consists principally of two structurally conserved surfaces located in the N-terminal domain: a polar binding pocket derived from three ß-strand elements of the ß-barrel domain and a hydrophobic reverse turn. The binding pocket of SEB contains residues E67, Y89 and Y115, and binds K39 of the DRa subunit. For SEA, the binding interface with the DR molecule is modeled to contain a similar binding pocket consisting of residues D70, Y92 and Y108. Mutation of residue Y89 in SEB or Y92 in SEA to alanine (**Fig. 2**) resulted in 100-fold reduction in DR1 binding. The substitution of alanine for Y89 in SEB and Y92 in SEA eliminates the hydrogen bond with K39 and disrupts packing interactions with adjacent protein residues. Modeling of the SEA mutant Y92A predicts an increase in solvent-accessible surface area for Y108 by a factor of two greater than the wild-type structure, allowing the formation of a hydrogen bond to the carboxylate group of D70 and thus disrupting key anchoring and recognition points for HLA-DR1. This effect is expected to be somewhat less in SEB due to the longer side chain at E67. Substitution of SEB Y115 with alanine also resulted in 100-fold reduction of binding. In contrast, the same replacement of Y108 in SEA yielded little to no change in DR1 binding (**Fig. 2a****),** suggesting the primary importance of SEA residues Y92 and D70 for stabilizing interactions with K39. The K39 side chain of DRα forms a strong ion-pair interaction with the SEB E67 carboxylate group and hydrogen bonds with the hydroxyl groups of Y89 and Y115. Substitution of SEB E67 by glutamine reduced binding affinity by 100-fold (**Fig. 2**), reflecting the replacement of the strong ionic bond with a weaker hydrogen bond. To optimize ion-pair interactions of the analogous SEA site, the shorter carboxylate side chain of D70 is predicted to shift K39 of DRα, weakening interactions with SEA Y108. The substitution of alanine for SEA Y108 is thus more easily accommodated than the homologous substitution of SEB Y115, without loss in DR1 binding.

Comparisons of the polar pocket with other bacterial superantigens were then made. SEC1-3 and SPE-A have conserved the critical DR1 binding-interface residues (**Fig. 1**), and share with SEB and SEA secondary structural elements of the DR1-binding surfaces. Asparagine in SED (N70) replaces the acidic side chain present in SEA, SEB, SPE-A and SEC1-3. Accordingly, for SED the salt bridge of the polar pocket is likely to be replaced by a hydrogen bond. Overall DR1 affinities for SED and SEA appeared to be equivalent (**Fig. 2b**), indicating that other interactions may compensate for the absence in SED of the ion-pair found in the other superantigens. For the case of TSST-1, mutating DRa residues K39 to serine or M36 to isoleucine has been shown to greatly reduce binding [Panina-Bordignon et al. (1992) J. Exp. Med. 176: 1779-1784]. Although primarily hydrophobic, the critical TSST-1 structural elements are conserved with the SEA and SEB polar binding pocket. SEB residues Y89 and Y115 are homologous to T69 and I85 in TSST-1, respectively, and SEB E67 is replaced by I46. These TSST-1 residues are positioned in a conserved ß-barrel domain found in both SEB and SEA. However, the TSST-1 site lacks polarity equivalent to SEB/SEA, and hydrogen bonding with the hydroxyl of TSST-1 residue T69 would require that DRα K39 extend 5 A into the pocket. TSST-1 binding utilizes an alternative strategy [Kim et al. (1994) Science 266: 1870-1874] consisting of hydrophobic contacts centered around residue I46, and potential ionic or hydrogen bonds bridging DRa residues E71 and K67 to R34 and D27, respectively, of TSST-1.

The hydrophobic region of the binding interface between SEB and the HLA-DR1 molecule consists of SEB residues 44-47, located in a large reverse turn connecting ß-strands 1 and 2 of SEB. These residues appear to make strong electrostatic interactions with DRα through their backbone atoms. The mutation of L45 to an arginine reduced overall HLA-DR1 binding greater than 100-fold (**Fig. 2b**), attributable to the less energetically favorable insertion of a highly charged residue into a hydrophobic depression on the DR1 molecule. The modeled DR1-SEA complex presents similar interactions with the SEA backbone atoms, with the exception of a glutamine (Q49) replacing SEB Y46. Mutation of L48 to glycine in SEA (homologous to L45 of SEB) has been reported to decrease T-cell responses. SEB L45 and the comparable L30 of TSST-1 are the most extensively buried residues in the DR1 interface. The leucine is conserved among the bacterial superantigens (**Fig. 3**) and may provide the necessary hydrophobic structural element for surface complementarity with DR1, consistent with the mutagenesis data for SEB and SEA.

The inventor has performed similar structure and function studies with TSST-1, SEC1 and SPE-A.

### EXAMPLE 3

### Molecular modelling and structural studies of staphylococcal and streptococcal superantigens: Some interactions of bacterial superantigens with MHC class II receptors are not conserved but are less important than the hydrophobic loop and polar pocket binding sites.

In determining the overall affinity of the superantigen for DR1, a contributory role is played by structural variations around the common binding motifs. A short, variable structured, disulfide-bonded loop is found in SEA and a homologous longer loop in SEB. The SEB residue Y94, contained within this loop, forms hydrophobic interactions with L60 and A61 of the DRα subunit. Replacement of Y94 with alanine partially inhibits DR1 binding (**Fig. 2a****,b**). An alanine is found in SEA (A97) and SEE at the position equivalent to SEB Y94, and mutating this residue in SEA to tyrosine results in disrupted instead of stabilized interactions with DR1 (**Fig. 2a**). Although the disulfide loops differ in structure between SEA and SEB, A97 apparently contributes to the DRα binding interface in a manner similar to Y94 of SEB. Because TSST-1 lacks a disulfide loop, similar contacts with DRa are replaced by interactions with β-strands of TSST-1. In a like manner, the absence of a salt bridge between the residues K39 of DRα and E67 of SED is apparently compensated for by stabilizing interactions occurring outside of the otherwise conserved dominant binding surfaces (**Fig. 2a****).**

### EXAMPLE 4

### Molecular modelling and structural studies of staphylococcal and streptococcal superantigens: Superantigen interactions with T-cell antigen receptors.

The amino acid residues in contact with TCR are located in regions of high sequence variability, presenting a unique surface for interaction with the TCR. Residues implicated in TCR interactions by mutagenesis of SEA and SEB reside in variable loop regions, while TSST-1 mutants that affect TCR binding are mainly located in an α helix [Acharya, R.K. et al. (1994) Nature 367, 94-97; Kim, J. et al. (1994) Science 266, 1870-1874]. Specifically, mutations that diminish T-cell receptor recognition of SEB include residues N23, Y61, and the homologous SEA N25 or Y64 **(****Fig. 2c****).** SEA residues S206 and N207 also control T-cell responses [Hudson, et al.(1992) J. Exp. Med. 177: 175-184]. Mutants of the polar binding pocket, SEA Y92A and SEB Y89A, equivalently reduced T-cell responses (**Fig. 2c**), reflecting the observed decreases in DR1-binding (**Fig. 2a****, b**). While supporting reduced T-cell responses, mutants SEA Y64A and SEB Y61A retained normal affinities for DR1 (**Fig. 2a****-c**).

### EXAMPLE 5

### Animal models for determining biological activity of bacterial superantigens: Mouse.

When compared to primates, mice are not very susceptible to the toxic effects of SE, and we therefore sought to increase sensitivity with a potentiating dose of lipopolysaccharide (LPS) from Gram-negative bacteria [Stiles et al. (1993) Infect. Immun. 61, 5333-5338]. There was no apparent effect in control animals injected with any of the SE (up to 30 µg/mouse) or LPS (150 µg/mouse) alone (Table 1). Incremental injections of LPS were also not lethal, when given in amounts up to 250 µg/mouse (data not shown). However, mice died between 24-48 h after SE and LPS were given to the same animal (Table 1). SEA was much more toxic than either SEB or SEC1 and the calculated LD50 (µg toxin/kg) of SEA, SEB, and SEC1 with 95% fiducial limits was 18.5 (6.5, 38.5), 789.0 (582.5, 1044.5), and 369.0 (197.5, 676.0), respectively.

**TABLE 1. Titration of SEA, SEB, and SEC₁ in the C57BL/6 mouse lethality assay**

| | % Lethality (no. of mice tested) with | | | |
|---|---|---|---|---|
| Stimulus^{a} | the following dose of SE, in micrograms/mouse^{b}: | | | |
| | 30 | 10 | 1 | 0.1 |
| SEA + LPS | 93 (15)^{b} | 85 (20) | 80 (15) | 20 (10) |
| SEB + LPS | 80 (15) | 27 (15) | 0 (15) | 0 (15) |
| SEC₁+ LPS | 80 (10) | 60 (10) | 10 (10) | 0 (10) |

| | | | | |
|---|---|---|---|---|
| ^{a}LPS was injected into each mouse (150ug) 4 h after the SE injection. Control mice injected with 150 ug of LPS (n=20) or 30 ug of SEA, SEB, or SEC1 (n=10) survived. ^{b}Results are from a combination of separate experiments with five mice per experiment. | | | | |

The role of MHC class I and class II molecules in SE toxicity, potentiated by LPS, was addressed by using transgenic, MHC-deficient mice (Table 2). Class II-deficient animals were unaffected by a dose of SE (30 µg) plus LPS (150 µg) that was lethal for 93% of wild-type and 30% of class I-deficient mice. Mononuclear cells from class II-deficient animals were not able to present SEA, as measured by proliferative responses. MHC class I-deficient cells were functional in supporting T-cell proliferation, but at levels <30% of the proliferative response supported by MHC-wild-type presenting cells (Table 3). Cell surface expression levels were normal, when compared to nontransgenic C57BL/6, for A^{b} in class I-deficient mice, and K^{b}/D^{b} in class II-deficient mice. The T-cell responses of MHC class I- or class II-deficient mice were essentially equivalent to wild-type when SEA was presented by mononuclear cells expressing both class I and II molecules (Table 3).

**TABLE 2. Lethality of SEA and SEB in C57BL/6 mice SEA and SEB in C57BL/6 mice**

| % Lethality (no. of mice tested) with Stimulus^{a} the following MHC class phenotype | | | |
|---|---|---|---|
| | I⁻II⁺ | I⁺II⁻ | I⁺II⁺ |
| SEA + LPS | 30 (10) | 0 (5) | 93 (15) |
| SEA + LPS | ND^{b} | 0 (5) | 80 (15) |
| SEA only | 0 (2) | 0 (2) | 0 (2) |
| SEB only | ND^{b} | 0 (2) | 0 (2) |
| LPS only | 0 (5) | 0 (5) | 0 (5) |

| | | | |
|---|---|---|---|
| ^{a} Mice were injected with 30 ug of SEA or SEB and, 4h later, with 150 ug of LPS, as indicated. Control mice were injected with only SEA, SEB, or LPS. ^{b} ND, not determined. | | | |

**Table 3. Mouse T-cell responses to SEA are MHC class II-dependent**

| | T-cell responses¹ | |
|---|---|---|
| T-cell/APC source³ | 0.1 µg/ml SEA | 1 µg/ml SEA |
| Wild-type C57/BL6 | | |
| mouse/autologous | 430,000 cpm² | 700,000 cpm |
| MHC class I | | |
| knock-out | 117,000 cpm | 167,000 cpm |
| C57/BL6 mouse/autologous | | |
| | | |
| MHC class II | | |
| knock-out | 8,000 cpm | 33,000 cpm |
| C57/BL6 mouse/autologous | | |
| | | |
| Wild-type C57/BL6 | | |
| mouse/wild-type | 305,000 cpm | 307,000 cpm |
| MHC class I | | |
| knock-out | 420,000 cpm | 445,000 cpm |
| C57/BL6 mouse/wild-type | | |
| MHC class II | | |
| knock-out C57/BL6 | 310,000 cpm | 322,000 cpm |
| mouse/wild-type | | |

| | | |
|---|---|---|
| ¹Cultures of mononuclear cells derived from mouse spleens, cultured for 3 d with the indicated amount of SEA. ²Data represent the mean of triplicate determinations (<10 SEM) of [³H]thymidine incorporation. ³Antigen presenting cells (APC) were isolated from spleens of the indicated mouse strain and added to cultures. | | |

The serum levels of TNFα IL-1α, IL-6, and IFNγ in mice injected with SEA, LPS, or SEA plus LPS were measured at various times following injection (**Fig. 4**). Compared to mice injected with either SEA or LPS alone, the serum levels of TNFα, IL-6, and IFNγ had increased 5-, 10-, and 15-fold, respectively, in animals given SEA plus LPS. SEA alone did not elicit any detectable increase of TNFα, IL-6, or IFNγ above background. In contrast to the other cytokines, IL-1α levels in mice injected with SEA plus LPS resulted in a simple additive effect.

Serum levels of TNFα, IL-6, and IFNγ were maximal 2-4 h after the LPS injection, but returned to normal by 10 h. The concentration of IL-1α in mice given SEA plus LPS had also peaked 2 h after the LPS injection, but stayed above background for the remaining determinations. Levels of IL-1α in mice given only LPS or SEA peaked at 4 and 6 h, respectively. Unlike profiles for other cytokines, the highest amount of IL-1α in mice injected with SEA and LPS corresponded to the peak stimulated by SEA, but not LPS.

This animal model was used in various stages of developing the inventions, as a means of assessing the physiological activity of mutated superantigens. Control animals survived the maximum dose of either SE or LPS, while mice receiving both agents died. Wild-type SEA was 43-fold more potent than SEB and 20-fold more potent than SEC1. By using BALB/c mice the toxicity of SEB was 10-20 fold higher. These data confirmed that the toxicity of SE was mainly exerted through a mechanism dependent on expression of MHC class II molecules and was linked to stimulated cytokine release. Thus this was a relevant preclinical model that could be used to predict human responses.

### EXAMPLE 6

### Animal models for determining biological activity of bacterial superantigens: Rhesus monkey

The physiological responses of the rhesus monkey to bacterial superantigens is probably identical to humans, with the exception of sensitivity [Bavari and Ulrich (1995) *Clin. Immunol.Immunopath.* 7**6:**248]. Generally SEB intoxicated monkeys developed gastrointestinal signs within 24 hours post-exposure. Clinical signs were mastication, anorexia, emesis and diarrhea. Following mild, brief, self-limiting gastrointestinal signs, monkeys had a variable period of up to 40 hours of clinical improvement. At approximately 48 hours post-exposure, intoxicated monkeys generally had an abrupt onset of rapidly progressive lethargy, dyspnea, and facial pallor. If given a lethal dose, death occurs within four hours of onset of symptoms. Only SEB has been used in challenges of rhesus monkeys to determine physiological/pathological effects. Human responses to bacterial superantigens are characterized by a rapid drop in blood pressure, elevated temperature, and multiple organ failure-the classical toxic shock syndrome (TSS). However, the respiratory route of exposure may involve some unique mechanisms. The profound hypotension characteristic of TSS is not observed, and respiratory involvement is rapid, unlike TSS. Fever, prominent after aerosol exposure, is generally not observed in cases of SEB ingestion.

### EXAMPLE 7

### Targeting receptor interactions to develop vaccines.

The SEA mutants Y92A, with reduced DR1 binding, and Y64A, with reduced TCR interactions, and K14E with wild-type (control) activity were used to determine the correct receptor to target for vaccine development. The binding of WT or mutant SEA was evaluated with the MHC class II expressing murine B-cell lymphoma cell line A20 (Table 4). The binding affinity of WT SEA to mouse MHC class II (H-2^{d}) molecules was lower than that observed with human MHC class II expressing cells , reflecting the reduced toxicity that bacterial SAgs exert in mice. WT SEA, Y64A and K14E all had the same relative affinity to mouse MHC class II molecules. Similar to the results obtained with human MHC class II molecules, the Y92A mutant exhibited substantially reduced binding to A20 cells (Table 4).

**Table 4. Biological activity of superantigen vaccines**

| | | | MHC classII | |
|---|---|---|---|---|
| toxin | T-cell | anergy¹ | binding² | T-cell response |
| SEA | | | | |
| wild type | | ++++ | +++ | +++ |
| TCR attenuated | | + | +++ | +/- |
| Y64A | | | | |
| MHC attenuated | | | +/- | +/- |
| Y92A | | | | |
| Control | | ++++ | +++ | +++ |
| K14E | | | | |

| | | | | |
|---|---|---|---|---|
| ¹Based on attenuation of T-cell response to wild-type SEA in mice immunized with the mutant or wild-type SEA. ²Binding to the mouse MHC class II+ A20 cells, measured by flow cytometry | | | | |

The effect of WT SEA or site-specific SEA mutants on splenic mononuclear cells obtained from nonimmunized C57BL/6 (H-2^{b}) mice is summarized in Table 4. Both WT SEA and the control mutant K14E were potent T cell activators, effective at minimal concentrations of 10 to 100 pg/mL. However, T-cell responses to Y92A were reduced at least 100-fold, compared to SEA wild type, while Y64A-stimulated responses were slightly higher than Y92A. These results confirmed that attenuation of superantigen binding to either MHC class II or TCR molecules resulted in dramatically reduced mouse T-cell proliferation. These results may indicate that the altered toxin may compete with wild type toxin for TCR binding.

SEA WT (10 LD50), site-specific SEA mutants (10 µg/mouse each) or LPS (150 µg/mice) injected alone were nonlethal to mice (Table 5). However, combining LPS with either WT SEA or mutant K14E resulted in 100% lethality. For those mice receiving both LPS and WT or K14E SEA, 80% were dead by 24 h and 100% by 48 h. In contrast, 100% of Y92A and 80% of Y64A injected mice (coadministered with LPS) survived. The average time to death for the 20% of mice that did not survive Y64A injection occurred at 48 to 72 h. These *in vivo* data correlated well with the results obtained with the lymphocyte cultures. It was concluded that the observed attenuation of toxicity in mice was a direct result of the reduced T-cell proliferation.

**Table 5. Biologic effect of wild type (WT) staphylococcal enterotoxin A (SEA) and SEA mutants.**

| Protein | No. live/total |
|---|---|
| WT | 0/10 |
| K14E | 0/10 |
| Y64A | 8/10 |
| Y92A | 10/10 |

| | |
|---|---|
| NOTE. Mice were given 10 LD₅₀ (10ug) of WT or mutant SEA. Lipopolysaccharide (150 ug/mouse) was injected 3 h later. | |

Having established that attenuation of receptor binding resulted in reduced toxicity, we next examined the immunogenicity of the SEA mutants. Mice were immunized with WT or mutant SEA. Control mice received adjuvant only or were left untreated. One week before challenge with WT SEA, mice were bled and serum antibody titers were determined for each group (Table 6). Mice immunized with the 2 µg of Y64A or Y92A had serum antibody titers of 1:5000 and 1:1000, respectively. Immunization with 2 µg of WT SEA or control mutant resulted in titers of 1:5,000 and 1:10,000, respectively. The highest immunizing dose (10 µg/mouse) was most effective for all animals, resulting in antibody titers which were greater than 1:10,000. All mice were challenged with 10 LD50 of WT SEA (potentiated with LPS). The survival data correlated well with the levels of serum antibodies in immunized mice. All mice that were vaccinated with 10 µg of Y64A or Y92A, survived the lethal challenge dose of WT SEA. Slightly less protection was afforded by the lower vaccination dose of mutant Y64A or Y92A. All mice immunized with both doses of WT SEA survived the lethal challenge with WT potentiated with LPS. Mice immunized with mutant K14E exhibited survivals of 100% and 80% for high and low vaccination doses, respectively. All nonimmunized or control mice that were vaccinated with adjuvant alone died when challenged with WT SEA and a potentiating dose of LPS.

**Table 6. Mice immunized with attenuated forms of staphylococcal enterotoxin A (SEA) produce high titers of neutralizing antibody.**

| Immunizing | Dose | Anti-SEA | |
|---|---|---|---|
| agent live/total | (ug/mouse) | antibody titer* | No. |
| WT | 2 | 10,000-50,000 | 10/10 |
| | 10 | 10,000-50,000 | 10/10 |
| K14E | 2 | 5,000-10,000 | 8/10 |
| | 10 | 10,000-50,000 | 10/10 |
| Y64A | 2 | 5,000-10,000 | 6/10 |
| | 10 | 10,000-50,000 | 10/10 |
| Y92A | 2 | 1,000-5,000 | 2/10 |
| | 10 | 10,000-50,000 | 10/10 |
| Adjuvant | | 50-100 | 0/10 |

| | | | |
|---|---|---|---|
| NOTE. Mice were given 10 LD₅₀ of wild type (WT) SEA challenge followed by potentiating dose of lipopolysaccharide (150 ug/mouse) 3 h later. *Reciprocal of serum dilution resulting in optical density reading four times above negative controls (wells containing either no SEA or no primary antibody). | | | |

### EXAMPLE 8

### Immune recognition of SAg mutants.

Bacterial SAgs induce clonal anergy of specific subsets of T cells in mice. It was possible that the loss of sensitivity to WT SEA among the mice vaccinated with the attenuated mutant forms represented a state of specific non-responsiveness instead of specific immunity. To address this issue, lymphocyte responses to SEA WT were measured with splenic mononuclear cells collected 2 weeks after the third immunization. As expected, lymphocytes from mice that were immunized with WT SEA or control SEA mutant showed little to no proliferation when incubated with the WT SAg. In contrast, lymphocytes obtained from control mice or those immunized with either Y64A or Y92A all responded vigorously to the WT SEA (Fig. 5). The TCRs used by T cells from the SEA-vaccinated mice were then characterized by flow cytometry. T cells from immunized or control mice were incubated with WT SEA in culture for 7 days, followed by a 5 day expansion in IL-2 containing medium. Distinct populations of activated TCR Vß11 positive cells were observed with T cells from mice immunized with Y92A and Y64A, representing 48% and 40% of T cells, respectively. However, Vß11 expressing cells obtained from SEA WT or K14E immunized mice were about 1% and 6% of the total T-cell population, respectively, suggesting that this subset was nonresponsive to restimulation with the WT SAg. T cells bearing Vß 17a, 3, 7, and 10b were unchanged for all mice. It was apparent that T-cell responses to both the TCR and MHC class II binding-attenuated SEA mutants were similar to each other, but differed from responses to control or WT molecules. These results suggested that an alternative, perhaps conventional antigen processing mechanism was functioning in presentation of the SAg mutants Y64A and Y92A.

### EXAMPLE 9

### Rhesus monkey immunizations with monovalent vaccines.

The SEA vaccine L48R, Y89A, D70R (A489270) and SEB vaccine Y89A, Y94A, L45R (B899445) were used to immunize rhesus monkeys. The animals received a total of three i.m. injections (10-20 µg/animal), given at monthly intervals. Rhesus monkeys that were injected with these vaccines had no detectable increase of serum cytokines and no apparent toxicity. The serological response of animals vaccinated with three doses of formalin-treated SEB toxoid (100 µg/injection) gave results comparable to one or two injections with B899445 (Table 7), suggesting that the recombinant vaccines were very immunogenic. Immunized rhesus monkeys survived a lethal challenge with >10 LD50 of wild-type SEB (Table 7, 8). Collectively, these results suggest that the engineered SEB vaccine is safe, highly antigenic and effective at protecting the immunized individual from lethal aerosol exposure to SEB.

**Table 7. Rhesus monkey antibody responses to vaccine B899445; One injection of B899445 outperforms three injections of SEB toxoid**

| | Antibody %Inhibition of Survival SEB | | |
|---|---|---|---|
| Vaccine¹/animal | # responses² | T-cell response³ | >20 x LD50 |
| | | | challenge⁴ preimmun |
| e sera /pooled | 0.161 | 5 | dead |
| toxoid/1 | 0.839 | 0 | dead |
| toxoid/2 | 0.893 | 34 | live |
| toxoid/3 | 1.308 | 57 | live |
| toxoid/4 | 1.447 | 55 | live |
| B899445/1 | 1.788 | 69 | live |
| B899445/2 | 0.78 | 49 | live |

| | | | |
|---|---|---|---|
| ¹Rhesus monkeys were immunized with one dose (20 µg injection) of B899445 vaccine or three doses of formalin-treated SEB toxoid (100 µg/injection) one month apart; both used Alum adjuvants. ²Sera were collected one month after the final injection. Antibody responses were determined by ELISA and the results are shown as mean optical densities of triplicate wells (± SEM). ³Rhesus monkey T cells, obtained from an untreated animal, were preincubated with diluted (1:70) serum from immunized monkeys and then cultured with wild type SEB. Data are shown as % of T cell responses, where serum of rhesus monkey injected with adjuvant only represented the 100% of response to wild type SEB. ⁴Rhesus monkeys were challenged by aerosol exposure and monitered for four days. | | | |

**Table 8. Engineered staphylococcal entero-toxin B vaccine efficacy in rhesus monkeys**

| Treatment¹ | Antibody titer² | Immune protection³ |
|---|---|---|
| Vaccine | | |
| with adjuvant | >10,000 | 100% |
| Adjuvant only | <50 | 0% |

| | | |
|---|---|---|
| ¹Rhesus monkeys (n=10) were injected i.m. with 10 µg of SEB vaccine with Alhydrogel adjuvant. A total of 3 immunizations, 1 month apart were given. Controls (n=2) received only Alhydrogel. ²Serum dilution resulting in optical density readings of four times above the negative control, consisting of no SEB or serum added to the wells. ³Immunized and control rhesus monkeys were challenged with >10 LD50 of wild-type staphylococcal enterotoxin B as an aerosol. | | |

Serum from monkeys that were immunized with the genetically attenuated vaccine inhibited T-lymphocyte responses to wild type SEB (Table 7) similarly or better than monkeys that received the SEB toxoid. Collectively, these results suggest that the recombinant SAg vaccines are safe, highly antigenic, and induce protective immunity.

Serum from B899445 immunized rhesus monkeys blocked human lymphocyte responses to wild-type superantigen when tested in *ex vivo* cultures (Table 7). These data again showed that the second and third injections of vaccine were approximately equivalent in stimulating neutralizing antibody responses. Normal T-cell responses to several superantigens, including the wild-type protein, were observed in immunized animals, indicating that no specific or generalized anergy occurred (**Fig. 6**).

### EXAMPLE 10

### A.Multivalent superantigen vaccines: Rhesus monkey immunizations.

Rhesus monkeys were immunized with a combined vaccine consisting of B899445 and A489270. Following the third injection, antibody recognition of wild-type bacterial superantigens was examined (**Fig.7**). High titers of anti-SEB, SEC1 and SEA antibodies were evident.

### B.Mouse immunizations.

Mice (BALB/c) were immunized with a combined vaccine consisting of SEA, SEB, SEC1 and TSST-1 (all wild-type). The antibody responses against each individual superantigen were assessed (Table 9). Antibodies were induced against each of the component antigens, providing sufficient levels to protect the mice from a lethal challenge of superantigen, potentiated with LPS. Although not shown in the Table, antibody responses against SPE-A were also observed. Mice were also immunized with individual superantigens and antibody responses against other superantigens were measured (Table 10). Each individual immunogen induced partial or complete protective antibody responses against all other superantigens tested.

**TABLE 9. Superantigen cross-reactivity of antibodies from mice immunized with individual bacterial superantigens**

| Immunizing¹ | Challenging² | ELISA³ | Neutralizing⁴ |
|---|---|---|---|
| Toxin | Antibody | Toxin | Titer |
| SEA | SEA | >1/25,000 | 100% |
| SEA | SEB | >1/25,000 | 100% |
| SEA | SEC1 | >1/25,000 | 100% |
| SEA | TSST1 | >1/10,000 | 100% |
| SEB | SEB | >1/25,000 | 100% |
| SEB | SEA | >1/10,000 | 100% |
| SEB | SEC1 | >1/2,500 | 100% |
| SEB | TSST1 | >1/10,000 | 100% |
| SEC1 | SEC1 | >1/10,000 | 100% |
| SEC1 | SEA | >1/10,000 | 100% |
| SEC1 | SEB | >1/25,000 | 100% |
| SEC1 | TSST1 | >1/10,000 | 100% |
| TSST1 | TSST1 | <1/10,000 | 100% |
| TSST1 | SEA | <1/1,000 | 50% |
| TSST1 | SEB | <1/1,000 | 40% |
| TSST1 | SEC1 | <1/1,000 | 40% |

| | | | |
|---|---|---|---|
| ¹Three injections with 20 ug of antigen (BALB/c mice). ²LPS-potentiated challenge with 10 LD₅₀s of superantigen. ³ELISA antibody response against an individual superantigen. ⁴Percent mice surviving an LPS-potentiated challenge (n=10). | | | |

**Table 10. Multivalent superantigen vaccine. Mouse immune responses.**

| Immunizing | Challenging | Antibody | |
|---|---|---|---|
| toxin¹ | toxin² | Titer³ | % survival |
| SE-A, B, C1, TSST-1 | all | N/A | 100% |
| " " | SEA | >25,000 | 100% |
| " " | SEB | >25,000 | 100% |
| " " | SEC1 | >25,000 | 100% |
| " " | TSST-1 | >6,400 | 100% |

| | | | |
|---|---|---|---|
| ¹Total of three injections, two weeks apart, in RIBI adjuvant. ²>10 X LD50, potentiated with *E. coli* lipopolysaccharide. ³Measured by ELISA. | | | |

### EXAMPLE 11

### Design of altered TSST-1 toxin vaccine, TST30.

A comprehensive study of the relationships of TSST-1 protein structure to receptor binding were undertaken to provide insight into the design of the vaccine TST30. We have discovered that TSST-1 interactions with the human MHC class II receptor, HLA-DR, are relatively weak and can be disrupted by altering only a single critical amino acid residue of the toxin. Site-directed mutagenesis of a gene encoding the toxin and expression of the new protein product in *E. coli* were then used to test the design of the vaccine. The TSST-1 gene used was contained within a fragment of DNA isolated by BglI restriction enzyme digestion of the gene isolated from a toxigenic strain of *Staphylococcus aureus* (AB259; Kreiswirth and Novick (1987) Mol. Gen. Genet. 208, 84-87). The sequence of this gene is identical to all currently known TSST-1 isolates of human origin. The wild-type TSST-1 gene can be readily cloned from a number of clinical S. aureus isolates. The DNA fragment containing the TSST-1 gene was isolated by agarose gel electrophoresis and ligated inot the prokaryotic expression vector pSE380 (Invitrogen Corp.). The DNA clone consisted of sequences encoding the leader peptide and the full length of the mature TSST-1 protein. This engineered vaccine is currently being evaluated to determine mouse and human T-cell reactivities in vitro, and lethality in mice. The TST30 vaccine consists of the following mutation introduced into the toxin molecule: leucine at amino acid residue 30 changed to arginine. Two other mutations, namely Asp27 to Ala and Ile46 to Ala have also been designed. The final vaccine may incorporate one or both of these additional mutations.

The binding interface between TSST-1 and HLA-DR consists of a large relatively flat surface located in the N-terminal domain. Leucine 30 protrudes from a reverse turn on the surface of TSST-1 and forms the major hydrophobic contact with the HLA-DR receptor molecule. Mutation of the single residue leucine 30 in TSST-1 to the charged amino acid side chain of arginine is predicted to disrupt this major contact with the receptor molecule, resulting in a significant reduction in DR1 binding. This mutant molecule should therefore have lost the toxin attributes of the wild-type molecule.

TST30 was expressed as a recombinant protein in E.coli, as either a periplasmically secreted protein or as a cytoplasmic product. Purification was achieved by immunoaffinity chromatography or preparative isoelectric focusing after an initial ion-exchange CM-Sepharose enrichment step. The method of purification was not critical to the performance of the vaccine. Lipopolysaccharide contaminants, resulting from expression in a Gram-negative bacterium, were readily removed (as determined by limulus assay) using a variety of standard methods. The final purified vaccine is not toxic to mice at levels equivalent to 10 LD₅₀ of the native TSST-1. No indicators of toxicity were found in surrogate assays of human T-cell stimulation.

Conclusive vaccine studies demonstrating that TST30 is highly antigenic and induces protective immunity are in progress in a mouse animal model. Mouse lethality is achieved at less than 1 µg/animal when a potentiating signal like lipopolysaccharide from Gram-negative bacteria (LPS) is provided. When coadministered with LPS, wild-type TSST-1 is 100% lethal to mice (10 LD₅₀). Mice receive three injections (two weeks between injections) of 20 ug/mouse in alhydrogel and protection against the lethal effects of 10 LD₅₀ of TSST-1 are assessed.

### EXAMPLE 12

### Design of altered SPEA toxin vaccine, SPEa42

The SPEa interactions with human MHC class II receptor, HLA-DR, are relatively weak and can be disrupted by altering only a single critical amino acid residue of the toxin. Site-directed mutagenesis of a gene encoding the toxin and expression of the new protein product in E.coli were then used to test the design of the vaccine. The SPEa gene used was clone from a SPEa-toxigenic strain of *Streptococcus* by using specific DNA oligonucleotide primers and the polymerase chain reaction method. The sequence of this gene is identicla to SPEa isolates of human origin known within the public domain. The DNA fragment containing the SPEa gene was isolated by agarose gel electrophoresis and ligated into a prokaryotic expression vector (pETx or pSE380). The DNA clone consisted of sequences encoding the leader peptide and the full length of the mature SPEa protein or SPEa42 without a leader sequence. We recognize that there are additional ways to express or produce the mature SPEa vaccine. The SPEa vaccine consists of the following mutation introduced into the toxin molecule: leucine at amino acid residue 42 changed to arginine.

The binding interface between SPEa and HLA-DR is predicted to consist of contacts located in the N-terminal domain that are conserved with other bacterial superantigens. Leucine 42 of SPEa is predicted to protrude from a reverse turn on the surface of SPEa and form a major hydrophobic contact with the HLA-DR receptor molecule. Mutation of the single residue leucine 42 in SPEa to the charged amino acid side chain of arginine is predicted to disrupt this major contact with the receptor molecule, resulting in a significant reduction in DR1 binding. This mutant molecule should therefore have lost the toxin attributes of the wild-type molecule.

SPEa42 was expressed as a recombinant protein in E.coli, as either a periplasmically secreted protein or as a cytoplasmic product. Purification was achieved by immunoaffinity chromatography or preparative isoelectric focusing after an initial ion-exchange CM-Sepharose enrichment step. The method of purification was not critical to the performance of the vaccine. Lipopolysaccharide contaminants, resulting from expression in a Gram-negative bacterium, were readily removed (as determined by limulus assay) using a variety of standard methods. The final purified vaccine is not toxic to mice at levels equivalent to 10 LD₅₀ of the native TSST-1. No indicators of toxicity were found in surrogate assays of human T-cell stimulation.

Conclusive vaccine studies demonstrating that SPEa42 is highly antigenic and induces protective immunity are in progress in a mouse animal model. Mouse lethality is achieved at less than 1 ug/animal when a potentiating signal like lipopolysaccharide from Gram-negative bacteria (LPS) is provided. When coadministered with LPS, wild-type SPEa is 100% lethal to mice (10 LD₅₀). Mice receive three injections (two weeks between injections) of 20 ug/mouse in alhydrogel and protection against the lethal effects of 10 LD₅₀ of SPEa are assessed

### EXAMPLE 13

### Design of altered superantigen toxin vaccine, SEC45

For Staphylococcal enterotoxin C1 (SEC1), the leucine at position 45 was changed to lysine (SEC45). This mutation is anticipated to prevent SEC1 from interacting with the MHC class II receptor by sterically blocking the hydrophobic loop (centered around leucine 45) from binding to the alpha chain of the receptor. SEC1 is more closely homologous to SEB than SEA or the other superantigen toxins. The presence of zinc in SEC1 may impart additional binding characteristics that allow, in some cases, this superantigen toxin to bind to T-cell antigen receptors without the required MHC class II molecule interactions. To circumvent the binding to T-cell antigen receptors, mutations of SEC1 residues N23 (changed to alanine), V91 (changed to lysine) are being performed.

## Claims

1. An isolated and purified DNA fragment which encodes an altered Staphylococcal enterotoxin A (SEA) superantigen toxin peptide wherein position 64 has been changed to alanine and one or more of positions 92, 70 and 48 has been changed to alanine, arginine, and/or arginine, respectively, whereby binding of the encoded altered toxin peptide to either the MHC class II or T cell antigen receptor is altered.

2. A DNA fragment according to claim 1, encoding said peptide wherein at least position 92 has been changed, to alanine.

3. A DNA fragment according to claim 1, encoding said peptide wherein at least position 70 has been changed, to arginine.

4. A DNA fragment according to claim 1, encoding said peptide wherein at least position 48 has been changed, to arginine.

5. A DNA fragment according to claim 1, encoding said peptide wherein the positions changed are 92, 70 and 48, wherein position 92 has been changed to alanine, position 70 has been changed to arginine and position 48 has been changed to arginine.

6. A DNA fragment according to claim 1, wherein said fragment encodes the amino acid sequence of SEQ ID NO:2 or the amino acid sequence of SEQ ID NO:4.

7. A DNA fragment according to claim 1, consisting of the sequence of SEQ ID NO:1 or SEQ ID NO:3.

8. A recombinant DNA construct comprising:
(i) a vector, and
(ii) an isolated and purified altered superantigen toxin DNA fragment according to any of claims 1 to 7.

9. A recombinant DNA construct according to claim 8, wherein said vector is an expression vector.

10. A host cell transformed with a recombinant DNA construct according to claim 8.

11. A host cell according to claim 10, wherein said cell is prokaryotic.

12. A method for producing altered superantigen toxin comprising culturing cells according to claim 10, under conditions such that said DNA fragment is expressed and said superantigen toxin is thereby produced, and isolating said superantigen toxin.

13. An isolated and purified altered SEA superantigen toxin peptide which has been altered such that position 64 has been changed to alanine and one or more of positions 92, 70 and 48 have been changed to alanine, arginine, and/or arginine, respectively, whereby binding of the encoded altered toxin peptide to either the MHC class II or T cell antigen receptor is altered.

14. A peptide according to claim 13, wherein at least position 92 has been changed, to alanine.

15. A peptide according to claim 13, wherein at least position 70 has been changed, to arginine.

16. A peptide according to claim 13, wherein at least position 48 has been changed, to arginine.

17. A peptide according to claim 13, wherein the positions changed are 92, 70 and 48, wherein position 92 has been changed to alanine, position 70 has been changed to arginine and position 48 has been changed to arginine.

18. A peptide according to claim 13, having the amino acid sequence of SEQ ID NO: 2 or SEQ ID NO: 4.

19. A method for the diagnosis of superantigen-associated bacterial infection comprising the steps of:
(i) contacting a sample from an individual suspected of having a superantigen-associated bacterial infection with altered superantigen toxin peptide according to any of claims 13 to 18; and
(ii) detecting the presence or absence of a superantigen-associated bacterial infection by detecting the presence or absence of a complex formed between the altered superantigen toxin peptide and antibodies specific therefor in the sample.

20. A superantigen toxin-associated infection diagnostic kit comprising an altered superantigen toxin peptide according to any of claims 13 to 18, and ancillary reagents suitable for use in detecting the presence or absence of antibodies against superantigen toxin in a mammalian sample.

21. A vaccine comprising an altered superantigen toxin peptide according to any of claims 13 to 18 effective for the production of antigenic and immunogenic response resulting in the protection of a mammal against superantigen-associated bacterial infection.

22. A vaccine according to claim 21 wherein said vaccine further comprises at least one other different altered superantigen toxin peptide chosen from the group consisting of SEB and SEA.

23. A vaccine according to claim 22 wherein said vaccine is bivalent and said other altered superantigen toxin peptide is an altered SEB peptide.

24. A vaccine according to claim 22 wherein said vaccine is multivalent.

## Patentansprüche

1. Isoliertes und gereinigtes DNA-Fragment, das für ein verändertes Staphylokokken-Enterotoxin-A- (SEA-) Superantigen-Toxin-Peptid codiert, wobei Position 64 zu Alanin verändert worden ist und eine oder mehrere der Positionen 92, 70 und 48 zu jeweils Alanin, Arginin und/oder Arginin verändert worden ist bzw. sind, wodurch Bindung des codierten veränderten Toxin-Peptids an entweder den MHC-Klasse-II- oder den T-Zell-Antigen-Rezeptor verändert wird.

2. DNA-Fragment nach Anspruch 1, das für das Peptid codiert, wobei zumindest Position 92 verändert worden ist, zu Alanin.

3. DNA-Fragment nach Anspruch 1, das für das Peptid codiert, wobei zumindest Position 70 verändert worden ist, zu Arginin.

4. DNA-Fragment nach Anspruch 1, das für das Peptid codiert, wobei zumindest Position 48 verändert worden ist, zu Arginin.

5. DNA-Fragment nach Anspruch 1, das für das Peptid codiert, wobei die veränderten Positionen 92, 70 und 48 sind, wobei Position 92 zu Alanin verändert worden ist, Position 70 zu Arginin verändert worden ist und Position 48 zu Arginin verändert worden ist.

6. DNA-Fragment nach Anspruch 1, wobei das Fragment für die Aminosäuresequenz von SEQ ID NO: 2 oder die Aminosäuresequenz von SEQ ID NO: 4 codiert.

7. DNA-Fragment nach Anspruch 1, das aus der Sequenz von SEQ ID NO: 1 oder SEQ ID NO: 3 besteht.

8. Rekombinantes DNA-Konstrukt, umfassend:
(i) einen Vektor, und
(ii) ein isoliertes und gereinigtes verändertes Superantigen-Toxin-DNA-Fragment nach einem der Ansprüche 1 bis 7.

9. Rekombinantes DNA-Konstrukt nach Anspruch 8, wobei der Vektor ein Expressionsvektor ist.

10. Wirtszelle, transformiert mit einem rekombinanten DNA-Konstrukt nach Anspruch 8.

11. Wirtszelle nach Anspruch 10, wobei die Zelle prokaryotisch ist.

12. Verfahren zur Produktion von verändertem Superantigen-Toxin, umfassend Kultivieren von Zellen nach Anspruch 10, unter solchen Bedingungen, dass das DNA-Fragment exprimiert und das Superantigen-Toxin dadurch produziert wird, und Isolieren des Superantigen-Toxins.

13. Isoliertes und gereinigtes verändertes SEA-Superantigen-Toxin-Peptid, das so verändert worden ist, dass Position 64 zu Alanin verändert worden ist und eine oder mehrere der Positionen 92, 70 und 48 zu jeweils Alanin, Arginin und/oder Arginin verändert worden ist bzw. sind, wodurch Bindung des codierten veränderten Toxin-Peptids an entweder den MHC-Klasse-II- oder den T-Zell-Antigen-Rezeptor verändert wird.

14. Peptid nach Anspruch 13, wobei zumindest Position 92 verändert worden ist, zu Alanin.

15. Peptid nach Anspruch 13, wobei zumindest Position 70 verändert worden ist, zu Arginin.

16. Peptid nach Anspruch 13, wobei zumindest Position 48 verändert worden ist, zu Arginin.

17. Peptid nach Anspruch 13, wobei die veränderten Positionen 92, 70 und 48 sind, wobei Position 92 zu Alanin verändert worden ist, Position 70 zu Arginin verändert worden ist und Position 48 zu Arginin verändert worden ist.

18. Peptid nach Anspruch 13, das die Aminosäuresequenz von SEQ ID NO: 2 oder SEQ ID NO: 4 aufweist.

19. Verfahren zur Diagnose von Superantigen-assoziierter bakterieller Infektion, umfassend die Schritte:
(i) In-Kontakt-Bringen einer Probe von einem Individuum, bei dem Verdacht auf eine Superantigen-assoziierte bakterielle Infektion besteht, mit verändertem Superantigen-Toxin-Peptid nach einem der Ansprüche 13 bis 18; und
(ii) Nachweisen des Vorliegens oder Nichtvorliegens einer Superantigenassoziierten bakteriellen Infektion durch Nachweisen des Vorliegens oder Nichtvorliegens eines Komplexes, der zwischen dem veränderten Superantigen-Toxin-Peptid und dafür spezifischen Antikörpern gebildet wird, in der Probe.

20. Diagnostisches Kit für Superantigen-Toxin-assoziierte Infektion, umfassend ein verändertes Superantigen-Toxin-Peptid nach einem der Ansprüche 13 bis 18, und zusätzliche Reagenzien, die sich zur Verwendung beim Nachweisen des Vorliegens oder Nichtvorliegens von Antikörpern gegen Superantigen-Toxin in einer Säugerprobe eignen.

21. Impfstoff, umfassend ein verändertes Superantigen-Toxin-Peptid nach einem der Ansprüche 13 bis 18, das für die Produktion einer antigenen und immunogenen Antwort wirksam ist, die im Schutz eines Säugers gegen Superantigen-assoziierte bakterielle Infektion resultiert.

22. Impfstoff nach Anspruch 21, wobei der Impfstoff weiterhin zumindest ein anderes verschiedenes verändertes Superantigen-Toxin-Peptid, ausgewählt aus der aus SEB und SEA bestehenden Gruppe, umfasst.

23. Impfstoff nach Anspruch 22, wobei der Impfstoff bivalent ist und das andere veränderte Superantigen-Toxin-Peptid ein verändertes SEB-Peptid ist.

24. Impfstoff nach Anspruch 22, wobei der Impfstoff multivalent ist.

## Revendications

1. Fragment d'ADN isolé et purifié qui code un peptide de toxine superantigénique de l'entérotoxine A de staphylocoque (SEA) modifié dans lequel la position 64 a été changée en alanine et une ou plusieurs des positions 92, 70 et 48 ont été changées en alanine, arginine et/ou arginine, respectivement, en vertu de quoi la liaison du peptide de toxine codé modifié soit au MHC de classe II soit au récepteur d'antigènes des cellules T est modifiée.

2. Fragment d'ADN selon la revendication 1, codant ledit peptide dans lequel la position 92 au moins a été changée en alanine.

3. Fragment d'ADN selon la revendication 1, codant ledit peptide dans lequel la position 70 au moins a été changée en arginine.

4. Fragment d'ADN selon la revendication 1, codant ledit peptide dans lequel la position 48 au moins a été changée en arginine.

5. Fragment d'ADN selon la revendication 1, codant ledit peptide dans lequel les positions changées sont les 92, 70 et 48, dans lequel la position 92 a été changée en alanine, la position 70 a été changée en arginine et la position 48 a été changée en arginine.

6. Fragment d'ADN selon la revendication 1, où ledit fragment code la séquence d'acides aminés de la SEQ ID NO: 2 ou la séquence d'acides aminés de la SEQ ID NO: 4.

7. Fragment d'ADN selon la revendication 1, consistant en la séquence de la SEQ ID NO: 1 ou de la SEQ ID NO: 3.

8. Produit de synthèse d'ADN recombinant, comprenant:
(i) un vecteur, et
(ii) un fragment d'ADN isolé et purifié de toxine superantigénique modifiée selon l'une quelconque des revendications 1 à 7.

9. Produit de synthèse d'ADN recombinant selon la revendication 8, dans lequel ledit vecteur est un vecteur d'expression.

10. Cellule hôte transformée avec un produit de synthèse d'ADN recombinant selon la revendication 8.

11. Cellule hôte selon la revendication 10, où ladite cellule est procaryote.

12. Procédé de production de toxine superantigénique modifiée comprenant cultiver des cellules selon la revendication 10, dans des conditions dans lesquelles ledit fragment d'ADN est exprimé et ladite toxine superantigénique est ainsi produite, et isoler ladite toxine superantigénique.

13. Peptide de toxine superantigénique SEA isolé et purifié modifié qui a été modifié de telle sorte que la position 64 a été changée en alanine et une ou plusieurs des positions 92, 70 et 48 ont été changées en alanine, arginine, et/ou arginine, respectivement, en vertu de quoi la liaison du peptide de toxine codé modifié soit au MHC de classe II soit au récepteur d'antigènes des cellules T est modifiée.

14. Peptide selon la revendication 13, dans lequel la position 92 au moins a été changée en alanine.

15. Peptide selon la revendication 13, dans lequel la position 70 au moins a été changée en arginine.

16. Peptide selon la revendication 13, dans lequel la position 48 au moins a été changée en arginine.

17. Peptide selon la revendication 13, dans lequel les positions changées sont les 92, 70 et 48, dans lequel la position 92 a été changée en alanine, la position 70 a été changée en arginine et la position 48 a été changée en arginine.

18. Peptide selon la revendication 13, ayant la séquence d'acides aminés de la SEQ ID NO: 2 ou de la SEQ ID NO: 4.

19. Procédé de diagnostic d'infection bactérienne associée à un superantigène, comprenant les étapes consistant en:
(i) mettre un échantillon provenant d'un individu soupçonné avoir une infection bactérienne associée à un superantigène en contact avec un peptide de toxine superantigénique modifié selon l'une quelconque des revendications 13 à 18; et
(ii) détecter la présence ou l'absence d'une infection bactérienne associée à un superantigène en détectant la présence ou l'absence d'un complexe formé entre le peptide de toxine superantigénique modifié et des anticorps spécifiques pour celui-ci dans l'échantillon.

20. Kit de diagnostic d'une infection associée à une toxine superantigénique comprenant un peptide de toxine superantigénique modifié selon l'une quelconque des revendications 13 à 18, et des réactifs auxiliaires convenant à être utilisés pour détecter la présence ou l'absence d'anticorps contre une toxine superantigénique dans un échantillon mammalien.

21. Vaccin comprenant un peptide de toxine superantigénique modifié selon l'une quelconque des revendications 13 à 18, effectif pour produire une réponse antigénique et immunogène résultant en la protection d'un mammifère contre une infection bactérienne associée à un superantigène.

22. Vaccin selon la revendication 21, où ledit vaccin comprend en outre au moins un peptide de toxine superantigénique différent modifié choisi parmi le groupe consistant en SEB et SEA.

23. Vaccin selon la revendication 22, où ledit vaccin est bivalent et l'autre dit peptide de toxine superantigénique modifié est un peptide SEB modifié.

24. Vaccin selon la revendication 22, où ledit vaccin est multivalent.


### SEQUENCE LISTING

(1) GENERAL INFORMATION:
   (i) APPLICANT:
      (A) Walter Reed Army Institute of Research
      (b) Department of the Army
      (c) Washington DC
      (d) DC 20307
      (e) USA
   (ii) TITLE OF INVENTION: Bacterial Superantigen Vaccines
   (iii) NUMBER OF SEQUENCES:25
   (iv) COMPUTER READABLE FORM:
      (A) MEDIUM TYPE: Floppy disk
      (B) COMPUTER: Dell PC
      (C) OPERATING SYSTEM: Windows
      (D) SOFTWARE: Microsoft Word 6.0
   (vi) CURRENT APPLICATION DATA:
      (A) APPLICATION NUMBER: WO 00/09154
      (B) FILING DATE: 13 August 1998
(2) INFORMATION FOR SEQUENCE ID NO:1:
   (i) SEQUENCE CHARACTERISTICS:
      (A) LENGTH: 830
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:1:
   ATGAAAAAAA CAGCATTTAC ATTACTTTTA TTCATTGCCC   40
   TAACGTTGAC AACAAGTCCA CTTGTAAATG GTAGCGAGAA   80
   AAGCGAAGAA ATAAATGAAA AAGATTTGCG AAAAAAGTCT  120
   GAATTGCAGG GAACAGCTTT AGGCAATCTT AAACAAATCT  160
   ATTATTACAA TGAAAAAGCT AAAACTGAAA ATAAAGAGAG  200
   TCACGATCAA TTTCGACAGC ATACTATATT GTTTAAAGGC  240
   TTTTTTACAG ATCATTCGTG GTATAACGAT TTATTAGTAC  280
   GTTTTGATTC AAAGGATATT GTTGATAAAT ATAAAGGGAA  320
   AAAAGTAGAC TTGTATGGTG CTTATGCTGG TTATCAATGT  360
   GCGGGTGGTA CACCAAACAA AACAGCTTGT ATGTATGGTG  400
   GTGTAACGTT ACATGATAAT AATCGATTGA CCGAAGAGAA  440
   AAAAGTGCCG ATCAATTTAT GGCTAGACGG TAAACAAAAT  480
   ACAGTACCTT TGGAAACGGT TAAAACGAAT AAGAAAAATG  520
   TAACTGTTCA GGAGTTGGAT CTTCAAGCAA GACGTTATTT  560
   ACAGGAAAAA TATAATTTAT ATAACTCTGA TGTTTTTGAT  600
   GGGAAGGTTC AGAGGGGATT AATCGTGTTT CATACTTCTA  640
   CAGAACCTTC GGTTAATTAC GATTTATTTG GTGCTCAAGG  680
   ACAGTATTCA AATACACTAT TAAGAATATA TAGAGATAAT  720
   AAAACGATTA ACTCTGAAAA CATGCATATT GATATATATT  760
   TATATACAAG TTAAACATGG TAGTTTTGAC CAACGTAATG  800
   TTCAGATTAT TATGAACCGA GAATAATCTA             830
(3) INFORMATION FOR SEQUENCE ID NO:2:
   (i) SEQUENCE CHARACTERISTICS:
      (A) LENGTH: 257
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:2:
   Met Lys Lys Thr Ala Phe Thr Leu Leu Leu
   1               5                   10
   Phe Ile Ala Leu Thr Leu Thr Thr Ser Pro
                   15                  20
   Leu Val Asn Gly Ser Glu Lys Ser Glu Glu
                   25                  30
   Ile Asn Glu Lys Asp Leu Arg Lys Lys Ser
                   35                  40
   Glu Leu Gln Gly Thr Ala Leu Gly Asn Leu
                   45                  50
   Lys Gln Ile Tyr Tyr Tyr Asn Glu Lys Ala
                   55                  60
   Lys Thr Glu Asn Lys Glu Ser His Asp Gln
                   65                  70
   Phe Arg Gln His Thr Ile Leu Phe Lys Gly
                   75                  80
   Phe Phe Thr Asp His Ser Trp Tyr Asn Asp
                   85                  90
   Leu Leu Val Arg Phe Asp Ser Lys Asp Ile
                   95                  100
   Val Asp Lys Tyr Lys Gly Lys Lys Val Asp
                   105                 110
   Leu Tyr Gly Ala Tyr Ala Gly Tyr Gln Cys
                   115                 120
   Ala Gly Gly Thr Pro Asn Lys Thr Ala Cys
                   125                 130
   Met Tyr Gly Gly Val Thr Leu His Asp Asn
                   135                 140
   Asn Arg Leu Thr Glu Glu Lys Lys Val Pro
                   145                 150
   Ile Asn Leu Trp Leu Asp Gly Lys Gln Asn
                   155                 160
   Thr Val Pro Leu Glu Thr Val Lys Thr Asn
                   165                 170
   Lys Lys Asn Val Thr Val Gln Glu Leu Asp
                   175                 180
   Leu Gln Ala Arg Arg Tyr Leu Gln Glu Lys
                   185                 190
   Tyr Asn Leu Tyr Asn Ser Asp Val Phe Asp
                   195                 200
   Gly Lys Val Gln Arg Gly Leu Ile Val Phe
                   205                 210
   His Thr Ser Thr Glu Pro Ser Val Asn Tyr
                   215                 220
   Asp Leu Phe Gly Ala Gln Gly Gln Tyr Ser
                   225                 230
   Asn Thr Leu leu Arg Ile Tyr Arg Asp Asn
                   235                 240
   Lys Thr Ile Asn Ser Glu Asn Met His Ile
                   245                 250
   Asp Ile Tyr Leu Tyr Thr Ser
                   255
(4) INFORMATION FOR SEQUENCE ID NO:3:
   (i) SEQUENCE CHARACTERISTICS:
      (A) LENGTH: 757
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:3:
   ATGAGAAAAG CGAAGAAATA AATGAAAAAG ATTTGCGAAA    40
   AAAGTCTGAA TTGCAGGGAA CAGCTTTAGG CAATCTTAAA    80
   CAAATCTATT ATTACAATGA AAAAGCTAAA ACTGAAAATA   120
   AAGAGAGTCA CGATCAATTT CGACAGCATA CTATATTGTT   160
   TAAAGGCTTT TTTACAGATC ATTCGTGGTA TAACGATTTA   200
   TTAGTACGTT TTGATTCAAA GGATATTGTT GATAAATATA   240
   AAGGGAAAAA AGTAGACTTG TATGGTGCTT ATGCTGGTTA   280
   TCAATGTGCG GGTGGTACAC CAAACAAAAC AGCTTGTATG   320
   TATGGTGGTG TAACGTTACA TGATAATAAT CGATTGACCG   360
   AAGAGAAAAA AGTGCCGATC AATTTATGGC TAGACGGTAA   400
   ACAAAATACA GTACCTTTGG AAACGGTTAA AACGAATAAG   440
   AAAAATGTAA CTGTTCAGGA GTTGGATCTT CAAGCAAGAC   480
   GTTATTTACA GGAAAAATAT AATTTATATA ACTCTGATGT   520
   TTTTGATGGG AAGGTTCAGA GGGGATTAAT CGTGTTTCAT   560
   ACTTCTACAG AACCTTCGGT TAATTACGAT TTATTTGGTG   600
   CTCAAGGACA GTATTCAAAT ACACTATTAA GAATATATAG   640
   AGATAATAAA ACGATTAACT CTGAAAACAT GCATATTGAT   680
   ATATATTTAT ATACAAGTTA AACATGGTAG TTTTGACCAA   720
   CGTAATGTTC AGATTATTAT GAACCGAGAA TAATCTA      757
(5) INFORMATION FOR SEQUENCE ID NO:4:
   (i) SEQUENCE CHARACTERISTICS:
      (A) LENGTH:233
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:4:
   Met Glu Lys Ser Glu Glu Ile Asn Glu Lys
                   5                   10
   Asp Leu Arg Lys Lys Ser Glu Leu Gln Gly
                   15                  20
   Thr Ala Leu Gly Asn Leu Lys Gln Ile Tyr
                   25                  30
   Tyr Tyr Asn Glu Lys Ala Lys Thr Glu Asn
                   35                  40
   Lys Glu Ser His Asp Gln Phe Arg Gln His
                   45                  50
   Thr Ile Leu Phe Lys Gly Phe Phe Thr Asp
                   55                  60
   His Ser Trp Tyr Asn Asp Leu Leu Val Arg
                   65                  70
   Phe Asp Ser Lys Asp Ile Val Asp Lys Tyr
                   75                  80
   Lys Gly Lys Lys Val Asp Leu Tyr Gly Ala
                   85                  90
   Tyr Ala Gly Tyr Gln Cys Ala Gly Gly Thr
                   95                  100
   Pro Asn Lys Thr Ala Cys Met Tyr Gly Gly
                   105                 110
   Val Thr Leu His Asp Asn Asn Arg Leu Thr
                   115                 120
   Glu Glu Lys Lys Val Pro Ile Asn Leu Trp
                   125                 130
   Leu Asp Gly Lys Gln Asn Thr Val Pro Leu
                   135                 140
   Glu Thr Val Lys Thr Asn Lys Lys Asn Val
                   145                 150
   Thr Val Gln Glu Leu Asp Leu Gln Ala Arg
                   155                 160
   Arg Tyr Leu Gln Glu Lys Tyr Asn Leu Tyr
                   165                 170
   Asn Ser Asp Val Phe Asp Gly Lys Val Gln
                   175                 180
   Arg Gly Leu Ile Val Phe His Thr Ser Thr
                   185                 190
   Glu Pro Ser Val Asn Tyr Asp Leu Phe Gly
                   195                 200
   Ala Gln Gly Gln Tyr Ser Asn Thr Leu Leu
                   205                 210
   Arg Ile Tyr Arg Asp Asn Lys Thr Ile Asn
                   215                 220
   Ser Glu Asn Met His Ile Asp Ile Tyr Leu
                   225                 230
   Tyr Thr Ser
(6) INFORMATION FOR SEQUENCE ID NO:5:
   (i) SEQUENCE CHARACTERISTICS:
      (A) LENGTH: 1712
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:5:
   GAACTAGGTA GAAAAATAAT TATGAGAAAA CACTATGTTG     40
   TTAAAGATGT TTTCGTATAT AAGTTTAGGT GATGTATAGT     80
   TACTTAATTT TAAAAGCATA ACTTAATTAA TATAAATAAC    120
   ATGAGATTAT TAAATATAAT TAAGTTTCTT TTAATGTTTT    160
   TTTAATTGAA TATTTAAGAT TATAACATAT ATTTAAAGTG    200
   TATCTAGATA CTTTTTGGGA ATGTTGGATA AAGGAGATAA    240
   AAAATGTATA AGAGATTATT TATTTCACAT GTAATTTTGA    280
   TATTCGCACT GATATTAGTT ATTTCTACAC CCAACGTTTT    320
   AGCAGAGAGT CAACCAGATC CTAAACCAGA TGAGTTGCAC    360
   AAATCGAGTA AATTCACTGG TTTGATGGAA GATATGAAAG    400
   TTTTGTATGA TGATAATCAT GTATCAGCAA TAAACGTTAA    440
   ATCTATAGAT CAATTTCTAT ACTTTGACTT AATATATTCT    480
   ATTAAGGACA CTAAGTTAGG GGATTATGAT AATGTTCGAG    520
   TCGAATTTAA AAACAAAGAT TTAGCTGATA AATACAAAGA    560
   TAAATACGTA GATGTGTTTG GAGCTAATTA TTATTATCAA    600
   TGTTATTTTT CTAAAAAAAC GAATGATATT AATTCGCATC    640
   AAACTGACAA ACGAAAAACT TGTATGTATG GTGGTGTAAC    680
   TGAGCATAAT GGAAACCAAT TAGATAAATA TAGAAGTATT    720
   ACTGTTCGGG TATTTGAAGA TGGTAAAAAT TTATTATCTT    760
   TTGACGTACA AACTAATAAG AAAAAGGTGA CTGCTCAAGA    800
   ATTAGATTAC CTAACTCGTC ACTATTTGGT GAAAAATAAA    840
   AAACTCTATG AATTTAACAA CTCGCCTTAT GAAACGGGAT    880
   ATATTAAATT TATAGAAAAT GAGAATAGCT TTTGGTATGA    920
   CATGATGCCT GCACCAGGAG ATAAATTTGC CCAATCTAAA    960
   TATTTAATGA TGTACAATGA CAATAAAATG GTTGATTCTA   1000
   AAGATGTGAA GATTGAAGTT TATCTTACGA CAAAGAAAAA   1040
   GTGAAATTAT ATTTTAGAAA AGTAAATATG AAGAGTTAGT   1080
   AATTAAGGCA GGCACTTATA GAGTACCTGC CTTTTCTAAT   1120
   ATTATTTAGT TATAGTTATT TTTGTTATAT CTCTCTGATT   1160
   TAGCATTAAC CCCTTGTTGC CATTATAGTT TTCACCAACT   1200
   TTAGCTGAAA TTGGGGGATC ATTTTTATCT TTACTATGGA   1240
   TAGTTACTGT GTCGCCGTTT TTAACGATTT GTTTCTCTTT   1280
   TAATTTGTCA GTTAATTTTT TCCATGCATC ATTTGCGTCA   1320
   AACCTATTTC CATTTGGATT TATTCTTGAC AAATCAATTC   1360
   TTTTAACACT ATCGGTATTA ATCGGCTTGT TATTAAAATT   1400
   ACTAAGTTCA TCTAAATCAG CTGTACCCGT AATACTACTT   1440
   TCGCCACCAT TATTTAAATT GTACGTAACA CCAACTGTCT   1480
   CATTTGCTGT TTTATCGATA ATATTTGCTT CTTTCAAAGC   1520
   ATCTCTTACA TTTTTCCATA AGTCTCTATC TGTTATTTCA   1560
   GAAGCCTTTG CAACGTTATT AATACCATTA TAATTTGAAG   1600
   AAGAATGAAA ACCTGAACCT ACTGTTGTTA AAACTAAAGC   1640
   ACTTGCTATC AATGTTCTTG TTAATAGTTT TTTATTCATT   1680
   TTATTTTCTC CTATAACTTA TTTGCAATCG AT           1712
(7) INFORMATION FOR SEQUENCE ID NO:6:
   (i) SEQUENCE CHARACTERISTICS:
      (A) LENGTH: 266
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:6:
   Met Tyr Lys Arg Leu Phe Ile Ser His Val
                   5                   10
   Ile Leu Ile Phe Ala Leu Ile Leu Val Ile
                   15                  20
   Ser Thr Pro Asn Val Leu Ala Glu Ser Gln
                   25                  30
   Pro Asp Pro Lys Pro Asp Glu Leu His Lys
                   35                  40
   Ser Ser Lys Phe Thr Gly Leu Met Glu Asp
                   45                  50
   Met Lys Val Leu Tyr Asp Asp Asn His Val
                   55                  60
   Ser Ala Ile Asn Val Lys Ser Ile Asp Gln
                   65                  70
   Phe Leu Tyr Phe Asp Leu Ile Tyr Ser Ile
                   75                  80
   Lys Asp Thr Lys Leu Gly Asp Tyr Asp Asn
                   85                  90
   Val Arg Val Glu Phe Lys Asn Lys Asp Leu
                   95                  100
   Ala Asp Lys Tyr Lys Asp Lys Tyr Val Asp
                   105                 110
   Val Phe Gly Ala Asn Tyr Tyr Tyr Gln Cys
                   115                 120
   Tyr Phe Ser Lys Lys Thr Asn Asp Ile Asn
                   125                 130
   Ser His Gln Thr Asp Lys Arg Lys Thr Cys
                   135                 140
   Met Tyr Gly Gly Val Thr Glu His Asn Gly
                   145                 150
   Asn Gln Leu Asp Lys Tyr Arg Ser Ile Thr
                   155                 160
   Val Arg Val Phe Glu Asp Gly Lys Asn Leu
                   165                 170
   Leu Ser Phe Asp Val Gln Thr Asn Lys Lys
                   175                 180
   Lys Val Thr Ala Gln Glu Leu Asp Tyr Leu
                   185                 190
   Thr Arg His Tyr Leu Val Lys Asn Lys Lys
                   195                 200
   Leu Tyr Glu Phe Asn Asn Ser Pro Tyr Glu
                   205                 210
   Thr Gly Tyr Ile Lys Phe Ile Glu Asn Glu
                   215                 220
   Asn Ser Phe Trp Tyr Asp Met Met Pro Ala
                   225                 230
   Pro Gly Asp Lys Phe Ala Gln Ser Lys Tyr
                   235                 240
   Leu Met Met Tyr Asn Asp Asn Lys Met Val
                   245                 250
   Asp Ser Lys Asp Val Lys Ile Glu Val Tyr
                   255                 260
   Leu Thr Thr Lys Lys Lys
                   265
(8) INFORMATION FOR SEQUENCE ID NO:7:
   (i) SEQUENCE CHARACTERISTICS:
      (A) LENGTH: 1712
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:7:
   GAACTAGGTA GAAAAATAAT TATGAGAAAA CACTATGTTG     40
   TTAAAGATGT TTTCGTATAT AAGTTTAGGT GATGTATAGT     80
   TACTTAATTT TAAAAGCATA ACTTAATTAA TATAAATAAC    120
   ATGAGATTAT TAAATATAAT TAAGTTTCTT TTAATGTTTT    160
   TTTAATTGAA TATTTAAGAT TATAACATAT ATTTAAAGTG    200
   TATCTAGATA CTTTTTGGGA ATGTTGGATA AAGGAGATAA    240
   AAAATGTATA AGAGATTATT TATTTCACAT GTAATTTTGA    280
   TATTCGCACT GATATTAGTT ATTTCTACAC CCAACGTTTT    320
   AGCAGAGAGT CAACCAGATC CTAAACCAGA TGAGTTGCAC    360
   AAATCGAGTA AATTCACTGG TTTGATGGAA AATATGAAAG    400
   TTTTGTATGA TGATAATCAT GTATCAGCAA TAAACGTTAA    440
   ATCTATAGAT CAATTTCGAT ACTTTGACTT AATATATTCT    480
   ATTAAGGACA CTAAGTTAGG GAATTATGAT AATGTTCGAG    520
   TCGAATTTAA AAACAAAGAT TTAGCTGATA AATACAAAGA    560
   TAAATACGTA GATGTGTTTG GAGCTAATGC TTATTATCAA    600
   TGTGCTTTTT CTAAAAAAAC GAATGATATT AATTCGCATC    640
   AAACTGACAA ACGAAAAACT TGTATGTATG GTGGTGTAAC    680
   TGAGCATAAT GGAAACCAAT TAGATAAATA TAGAAGTATT    720
   ACTGTTCGGG TATTTGAAGA TGGTAAAAAT TTATTATCTT    760
   TTGACGTACA AACTAATAAG AAAAAGGTGA CTGCTCAAGA    800
   ATTAGATTAC CTAACTCGTC ACTATTTGGT GAAAAATAAA    840
   AAACTCTATG AATTTAACAA CTCGCCTTAT GAAACGGGAT    880
   ATATTAAATT TATAGAAAAT GAGAATAGCT TTTGGTATGA    920
   CATGATGCCT GCACCAGGAG ATAAATTTGA CCAATCTAAA    960
   TATTTAATGA TGTACAATGA CAATAAAATG GTTGATTCTA   1000
   AAGATGTGAA GATTGAAGTT TATCTTACGA CAAAGAAAAA   1040
   GTGAAATTAT ATTTTAGAAA AGTAAATATG AAGAGTTAGT   1080
   AATTAAGGCA GGCACTTATA GAGTACCTGC CTTTTCTAAT   1120
   ATTATTTAGT TATAGTTATT TTTGTTATAT CTCTCTGATT   1160
   TAGCATTAAC CCCTTGTTGC CATTATAGTT TTCACCAACT   1200
   TTAGCTGAAA TTGGGGGATC ATTTTTATCT TTACTATGGA   1240
   TAGTTACTGT GTCGCCGTTT TTAACGATTT GTTTCTCTTT   1280
   TAATTTGTCA GTTAATTTTT TCCATGCATC ATTTGCGTCA   1320
   AACCTATTTC CATTTGGATT TATTCTTGAC AAATCAATTC   1360
   TTTTAACACT ATCGGTATTA ATCGGCTTGT TATTAAAATT   1400
   ACTAAGTTCA TCTAAATCAG CTGTACCCGT AATACTACTT   1440
   TCGCCACCAT TATTTAAATT GTACGTAACA CCAACTGTCT   1480
   CATTTGCTGT TTTATCGATA ATATTTGCTT CTTTCAAAGC   1520
   ATCTCTTACA TTTTTCCATA AGTCTCTATC TGTTATTTCA   1560
   GAAGCCTTTG CAACGTTATT AATACCATTA TAATTTGAAG   1600
   AAGAATGAAA ACCTGAACCT ACTGTTGTTA AAACTAAAGC   1640
   ACTTGCTATC AATGTTCTTG TTAATAGTTT TTTATTCATT   1680
   TTATTTTCTC CTATAACTTA TTTGCAATCG AT           1712
(9) INFORMATION FOR SEQUENCE ID NO:8:
   (i) SEQUENCE CHARACTERISTICS:
      (A) LENGTH: 266
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:8:
   Met Tyr Lys Arg Leu Phe Ile Ser His Val
                   5                   10
   Ile Leu Ile Phe Ala Leu Ile Leu Val Ile
                   15                  20
   Ser Thr Pro Asn Val Leu Ala Glu Ser Gln
                   25                  30
   Pro Asp Pro Lys Pro Asp Glu Leu His Lys
                   35                  40
   Ser Ser Lys Phe Thr Gly Leu Met Glu Asn
                   45                  50
   Met Lys Val Leu Tyr Asp Asp Asn His Val
                   55                  60
   Ser Ala Ile Asn Val Lys Ser Ile Asp Gln
                   65                  70
   Phe Arg Tyr Phe Asp Leu Ile Tyr Ser Ile
                   75                  80
   Lys Asp Thr Lys Leu Gly Asn Tyr Asp Asn
                   85                  90
   Val Arg Val Glu Phe Lys Asn Lys Asp Leu
                   95                  100
   Ala Asp Lys Tyr Lys Asp Lys Tyr Val Asp
                   105                 110
   Val Phe Gly Ala Asn Ala Tyr Tyr Gln Cys
                   115                 120
   Ala Phe Ser Lys Lys Thr Asn Asp Ile Asn
                   125                 130
   Ser His Gln Thr Asp Lys Arg Lys Thr Cys
                   135                 140
   Met Tyr Gly Gly Val Thr Glu His Asn Gly
                   145                 150
   Asn Gln Leu Asp Lys Tyr Arg Ser Ile Thr
                   155                 160
   Val Arg Val Phe Glu Asp Gly Lys Asn Leu
                   165                 170
   Leu Ser Phe Asp Val Gln Tyr Asn Lys Lys
                   175                 180
   Lys Val Thr Ala Gln Glu Leu Asp Tyr Leu
                   185                 190
   Thr Arg His Tyr Leu Val Lys Asn Lys Lys
                   195                 200
   Leu Tyr Glu Phe Asn Asn Ser Pro Tyr Glu
                   205                 210
   Thr Gly Tyr Ile Lys Phe Ile Glu Asn Glu
                   215                 220
   Asn Ser Phe Trp Tyr Asp Met Met Pro Ala
                   225                 230
   Pro Gly Asp Lys Phe Asp Gln Ser Lys Tyr
                   235                 240
   Leu Met Met Tyr Asn Asp Asn Lys Met Val
                   245                 250
   Asp Ser Lys Asp Val Lys Ile Glu Val Tyr
                   255                 260
   Leu Thr Thr Lys Lys Lys
                   265
(10) INFORMATION FOR SEQUENCE ID NO:9:
   (i) SEQUENCE CHARACTERISTICS:
      (A) LENGTH: 1388
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:9:
(11) INFORMATION FOR SEQUENCE ID NO:10:
   (i) SEQUENCE CHARACTERISTICS:
      (A) LENGTH: 239
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:10:
   Met Ser Gln Pro Asp Pro Lys Pro Asp Glu
                   5                   10
   Leu His Lys Ser Ser Lys Phe Thr Gly Leu
                   15                  20
   Met Glu Asn Met Lys Val Leu Tyr Asp Asp
                   25                  30
   Asn His Val Ser Ala Ile Asn Val Lys Ser
                   35                  40
   Ile Asp Gln Phe Arg Tyr Phe Asp Leu Ile
                   45                  50
   Tyr Ser Ile Lys Asp Thr Lys Leu Gly Asn
                   55                  60
   Tyr Asp Asn Val Arg Val Glu Phe Lys Asn
                   65                  70
   Lys Asp Leu Ala Asp Lys Tyr Lys Asp Lys
                   75                  80
   Tyr Val Asp Val Phe Gly Ala Asn Ala Tyr
                   85                  90
   Tyr Gln Cys Ala Phe Ser Lys Lys Thr Asn
                   95                  100
   Asp Ile Asn Ser His Gln Thr Asp Lys Arg
                   105                 110
   Lys Thr Cys Met Tyr Gly Gly Val Thr Glu
                   115                 120
   His Asn Gly Asn Gln Leu Asp Lys Tyr Arg
                   125                 130
   Ser Ile Thr Val Arg Val Phe Glu Asp Gly
                   135                 140
   Lys Asn Leu Leu Ser Phe Asp Val Gln Thr
                   145                 150
   Asn Lys Lys Lys Val Thr Ala Gln Glu Leu
                   155                 160
   Asp Tyr Leu Thr Arg His Tyr Leu Val Lys
                   165                 170
   Asn Lys Lys Leu Tyr Glu Phe Asn Asn Ser
                   175                 180
   Pro Tyr Glu Thr Gly Tyr Ile Lys Phe Ile
                   185                 190
   Glu Asn Glu Asn Ser Phe Trp Tyr Asp Met
                   195                 200
   Met Pro Ala Pro Gly Asp Lys Phe Asp Gln
                   205                 210
   Ser Lys Tyr Leu Met Met Tyr Asn Asp Asn
                   215                 220
   Lys Met Val Asp Ser Lys Asp Val Lys Ile
                   225                 230
   Glu Val Tyr Leu Thr Thr Lys Lys Lys
                   235
(12) INFORMATION FOR SEQUENCE ID NO:11:
   (i) SEQUENCE CHARACTERISTICS:
      (A) LENGTH: 731
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:11:
   TAAGGAGAAT TAAAAATGAA TAAAAAATTA CTAATGAATT   40
   TTTTTATCGT AAGCCCTTTG TTGCTTGCGA CAACTGCTAC   80
   AGATTTTACC CCTGTTCCCT TATCATCTAA TCAAATAATC  120
   AAAACTGCAA AAGCATCTAC AAACGATAAT ATAAAGGATT  160
   TGCTAGACTG GTATAGTAGT GGGTCTGACA CTTTTACAAA  200
   TAGTGAAGTT TTAGATAATT CCAGAGGATC TATGCGTATA  240
   AAAAACACAG ATGGCAGCAT CAGCTTGATA ATTTTTCCGA  280
   GTCCTTATTA TAGCCCTGCT TTTACAAAAG GGGAAAAAGT  320
   TGACTTAAAC ACAAAAAGAA CTAAAAAAAG CCAACATACT  360
   AGCGAAGGAA CTTATATCCA TTTCCAAATA AGTGGCGTTA  400
   CAAATACTGA AAAATTACCT ACTCCAATAG AACTACCTTT  440
   AAAAGTTAAG GTTCATGGTA AAGATAGCCC CTTAAAGTAT  480
   GGGCCAAAGT TCGATAAAAA ACAATTAGCT ATATCAACTT  520
   TAGACTTTGA AATTCGTCAT CAGCTAACTC AAATACATGG  560
   ATTATATCGT TCAAGCGATA AAACGGGTGG TTATTGGAAA  600
   ATAACAATGA ATGACGGATC CACATATCAA AGTGATTTAT  640
   CTAAAAAGTT TGAATACAAT ACTGAAAAAC CACCTATAAA  680
   TATTGATGAA ATAAAAACTA TAGAAGCAGA AATTAATTAA  720
   TTTACCACTT T                                 731
(13) INFORMATION FOR SEQUENCE ID NO:12:
   (i) SEQUENCE CHARACTERISTICS:
      (A) LENGTH: 234
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:12:
   Met Asn Lys Lys Leu Leu Met Asn Phe Phe
                    5                  10
   Ile Val Ser Pro Leu Leu Leu Ala Thr Thr
                   15                  20
   Ala Thr Asp Phe Thr Pro Val Pro Leu Ser
                   25                  30
   Ser Asn Gln Ile Ile Lys Thr Ala Lys Ala
                   35                  40
   Ser Thr Asn Asp Asn Ile Lys Asp Leu Leu
                   45                  50
   Asp Trp Tyr Ser Ser Gly Ser Asp Thr Phe
                   55                  60
   Thr Asn Ser Glu Val Leu Asp Asn Ser Arg
                   65                  70
   Gly Ser Met Arg Ile Lys Asn Thr Asp Gly
                   75                  80
   Ser Ile Ser Leu Ile Ile Phe Pro Ser Pro
                   85                  90
   Tyr Tyr Ser Pro Ala Phe Thr Lys Gly Glu
                   95                  100
   Lys Val Asp Leu Asn Thr Lys Arg Thr Lys
                   105                 110
   Lys Ser Gln His Thr Ser Glu Gly Thr Tyr
                   115                 120
   Ile His Phe Gln Ile Ser Gly Val Thr Asn
                   125                 130
   Thr Glu Lys Leu Pro Thr Pro Ile Glu Leu
                   135                 140
   Pro Leu Lys Val Lys Val His Gly Lys Asp
                   145                 150
   Ser Pro Leu Lys Tyr Gly Pro Lys Phe Asp
                   155                 160
   Lys Lys Gln Leu Ala Ile Ser Thr Leu Asp
                   165                 170
   Phe Glu Ile Arg His Gln Leu Thr Gln Ile
                   175                 180
   His Gly Leu Tyr Arg Ser Ser Asp Lys Thr
                   185                 190
   Gly Gly Tyr Trp Lys Ile Thr Met Asn Asp
                   195                 200
   Gly Ser Thr Tyr Gln Ser Asp Leu Ser Lys
                   205                 210
   Lys Phe Glu Tyr Asn Thr Glu Lys Pro Pro
                   215                 220
   Ile Asn Ile Asp Glu Ile Lys Thr Ile Glu
                   225                 230
   Ala Glu Ile Asn
(14) INFORMATION FOR SEQUENCE ID NO:13:
   (i) SEQUENCE CHARACTERISTICS:
      (A) LENGTH: 1095
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:13:
   ATCATTAAAT ATAATTAATT TTCTTTTAAT ATTTTTTTAA     40
   TTGAATATTT AAGATTATAA GATATATTTA AAGTGTATCT     80
   AGATACTTTT TGGGAATGTT GGATGAAGGA GATAAAAATG    120
   AATAAGAGTC GATTTATTTC ATGCGTAATT TTGATATTCG    160
   CACTTATACT AGTTCTTTTT ACACCCAACG TATTAGCAGA    200
   GAGCCAACCA GACCCTACGC CAGATGAGTT GCACAAAGCG    240
   AGTAAATTCA CTGGTTTGAT GGAAAATATG AAAGTTTTAT    280
   ATGATGATCA TTATGTATCA GCAACTAAAG TTAAGTCTGT    320
   AGATAAATTT AGGGCACATG ATTTAATTTA TAACATTAGT    360
   GATAAAAAAC TGAAAAATTA TGACAAAGTG AAAACAGAGT    400
   TATTAAATGA AGGTTTAGCA AAGAAGTACA AAGATGAAGT    440
   AGTTGATGTG TATGGATCAA ATTACTATGT AAACTGCTAT    480
   TTTTCATCCA AAGATAATGT AGGTAAAGTT ACAGGTGGCA    520
   AAACTTGTAT GTATGGAGGA ATAACAAAAC ATGAAGGAAA    560
   CCACTTTGAT AATGGGAACT TACAAAATGT ACTTATAAGA    600
   GTTTATGAAA ATAAAAGAAA CACAATTTCT TTTGAAGTGC    640
   AAACTGATAA GAAAAGTGTA ACAGCTCAAG AACTAGACAT    680
   AAAAGCTAGG AATTTTTTAA TTAATAAAAA AAATTTGTAT    720
   GAGTTTAACA GTTCACCATA TGAAACAGGA TATATAAAAT    760
   TTATTGAAAA TAACGGCAAT ACTTTTTGGT ATGATATGAT    800
   GCCTGCACCA GGCGATAAGT TTGACCAATC TAAATATTTA    840
   ATGATGTACA ACGACAATAA AACGGTTGAT TCTAAAAGTG    880
   TGAAGATAGA AGTCCACCTT ACAACAAAGA ATGGATAATG    920
   TTAATCCGAT TTTGATATAA AAAGTGAAAG TATTAGATAT    960
   ATTTGAAAGG TAAGTACTTC GGTGCTTGCC TTTTTAGGAT   1000
   GCATATATAT AGATTAAACC GCACTTCTAT ATTAATAGAA   1040
   AGTGCGGTTA TTTATACACT CAATCTAAAC TATAATAATT   1080
   GGAATCATCT TCAAA                              1095
(15) INFORMATION FOR SEQUENCE ID NO:14:
   (i) SEQUENCE CHARACTERISTICS:
      (A) LENGTH: 266
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:14:
   Met Asn Lys Ser Arg Phe Ile Ser Cys Val
                   5                   10
   Ile Leu Ile Phe Ala Leu Ile Leu Val Leu
                   15                  20
   Phe Thr Pro Asn Val Leu Ala Glu Ser Gln
                   25                  30
   Pro Asp Pro Thr Pro Asp Glu Leu His Lys
                   35                  40
   Ala Ser Lys Phe Thr Gly Leu Met Glu Asn
                   45                  50
   Met Lys Val Leu Tyr Asp Asp His Tyr Val
                   55                  60
   Ser Ala Thr Lys Val Lys Ser Val Asp Lys
                   65                  70
   Phe Arg Ala His Asp Leu Ile Tyr Asn Ile
                   75                  80
   Ser Asp Lys Lys Leu Lys Asn Tyr Asp Lys
                   85                  90
   Val Lys Thr Glu Leu Leu Asn Glu Gly Leu
                   95                  100
   Ala Lys Lys Tyr Lys Asp Glu Val Val Asp
                   105                 110
   Val Tyr Gly Ser Asn Tyr Tyr Val Asn Cys
                   115                 120
   Tyr Phe Ser Ser Lys Asp Asn Val Gly Lys
                   125                 130
   Val Thr Gly Gly Lys Thr Cys Met Tyr Gly
                   135                 140
   Gly Ile Thr Lys His Glu Gly Asn His Phe
                   145                 150
   Asp Asn Gly Asn Leu Gln Asn Val Leu Ile
                   155                 160
   Arg Val Tyr Glu Asn Lys Arg Asn Thr Ile
                   165                 170
   Ser Phe Glu Val Gln Thr Asp Lys Lys Ser
                   175                 180
   Val Thr Ala Gln Glu Leu Asp Ile Lys Ala
                   185                 190
   Arg Asn Phe Leu Ile Asn Lys Lys Asn Leu
                   195                 200
   Tyr Glu Phe Asn Ser Ser Phe Tyr Glu Thr
                   205                 210
   Gly Tyr Ile Lys Phe Ile Glu Asn Asn Gly
                   215                 220
   Asn Thr Phe Trp Tyr Asp Met Met Pro Ala
                   225                 230
   Pro Gly Asp Lys Phe Asp Gln Ser Lys Tyr
                   235                 240
   Leu Met Met Tyr Asn Asp Asn Lys Thr Val
                   245                 250
   Asp Ser Lys Ser Val Lys Ile Glu Val His
                   255                 260
   Leu Thr Thr Lys Asn Gly
                   265
(16) INFORMATION FOR SEQUENCE ID NO:15:
   (i) SEQUENCE CHARACTERISTICS:
      (A) LENGTH: 1837
      (B) TYPE: Nucleic Acid
      (C) STRANDEDNESS: Unknown
      (D) TOPOLOGY: Unknown
   (ii) Molecule type: DNA
   (xi) SEQUENCE DESCRIPTION: SEQ ID NO:15:
   TCATGTTTGA CAGCTTATCA TCGATAAGCT TACTTTTCGA 40
   ATCAGGTCTA TCCTTGAAAC AGGTGCAACA TAGATTAGGG 80
   CATGGAGATT TACCAGACAA CTATGAACGT ATATACTCAC 120
   ATCACGCAAT CGGCAATTGA TGACATTGGA ACTAAATTCA 160
   ATCAATTTGT TACTAACAAG CAACTAGATT GACAACTAAT 200
   TCTCAACAAA CGTTAATTTA ACAACATTCA AGTAACTCCC 240
   ACCAGCTCCA TCAATGCTTA CCGTAAGTAA TCATAACTTA 280
   CTAAAACCTT GTTACATCAA GGTTTTTTCT TTTTGTCTTG 320
   TTCATGAGTT ACCATAACTT TCTATATTAT TGACAACTAA 360
   ATTGACAACT CTTCAATTAT TTTTCTGTCT ACTCAAAGTT 400
   TTCTTCATTT GATATAGTCT AATTCCACCA TCACTTCTTC 440
   CACTCTCTCT ACCGTCACAA CTTCATCATC TCTCACTTTT 480
   TCGTGTGGTA ACACATAATC AAATATCTTT CCGTTTTTAC 520
   GCACTATCGC TACTGTGTCA CCTAAAATAT ACCCCTTATC 560
   AATCGCTTCT TTAAACTCAT CTATATATAA CATATTTCAT 600
   CCTCCTACCT ATCTATTCGT AAAAAGATAA AAATAACTAT 640
   TGTTTTTTTT GTTATTTTAT AATAAAATTA TTAATATAAG 680
   TTAATGTTTT TTAAAAATAT ACAATTTTAT TCTATTTATA      720
   GTTAGCTATT TTTTCATTGT TAGTAATATT GGTGAATTGT      760
   AATAACCTTT TTAAATCTAG AGGAGAACCC AGATATAAAA      800
   TGGAGGAATA TTAATGGAAA ACAATAAAAA AGTATTGAAG      840
   AAAATGGTAT TTTTTGTTTT AGTGACATTT CTTGGACTAA      880
   CAATCTCGCA AGAGGTATTT GCTCAACAAG ACCCCGATCC      920
   AAGCCAACTT CACAGATCTA GTTTAGTTAA AAACCTTCAA      960
   AATATATATT TTCTTTATGA GGGTGACCCT GTTACTCACG     1000
   AGAATGTGAA ATCTGTTGAT CAACTTAGAT CTCACGATTT     1040
   AATATATAAT GTTTCAGGGC CAAATTATGA TAAATTAAAA     1080
   ACTGAACTTA AGAACCAAGA GATGGCAACT TTATTTAAGG     1120
   ATAAAAACGT TGATATTTAT GGTGTAGAAT ATTACCATCT     1160
   CTGTTATTTA TGTGAAAATG CAGAAAGGAG TGCATGTATC     1200
   TACGGAGGGG TAACAAATCA TGAAGGGAAT CATTTAGAAA     1240
   TTCCTAAAAA GATAGTCGTT AAAGTATCAA TCGATGGTAT     1280
   CCAAAGCCTA TCATTTGATA TTGAAACAAA TAAAAAAATG     1320
   GTAACTGCTC AAGAATTAGA CTATAAAGTT AGAAAATATC     1360
   TTACAGATAA TAAGCAACTA TATACTAATG GACCTTCTAA     1400
   ATATGAAACT GGATATATAA AGTTCATACC TAAGAATAAA     1440
   GAAAGTTTTT GGTTTGATTT TTTCCCTGAA CCAGAATTTA     1480
   CTCAATCTAA ATATCTTATG ATATATAAAG ATAATGAAAC     1520
   GCTTGACTCA AACACAAGCC AAATTGAAGT CTACCTAACA     1560
   ACCAAGTAAC TTTTTGCTTT TGGCAACCTT ACCTACTGCT     1600
   GGATTTAGAA ATTTTATTGC AATTCTTTTA TTAATGTAAA     1640
   AACCGCTCAT TTGATGAGCG GTTTTGTCTT ATCTAAAGGA     1680
   GCTTTACCTC CTAATGCTGC AAAATTTTAA ATGTTGGATT     1720
   TTTGTATTTG TCTATTGTAT TTGATGGGTA ATCCCATTTT     1760
   TCGACAGACA TCGTCGTGCC ACCTCTAACA CCAAAATCAT     1800
   AGACAGGAGC TTGTAGCTTA GCAACTATTT TATCGTC        1837
(17) INFORMATION FOR SEQUENCE ID NO:16:
   (i) SEQUENCE CHARACTERISTICS:
      (A) LENGTH: 251
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:16:
   Met Glu Asn Asn Lys Lys Val Leu Lys Lys
   5 10
   Met Val Phe Phe Val Leu Val Thr Phe Leu
   15 20
   Gly Leu Thr Ile Ser Gln Glu Val Phe Ala
   25 30
   Gln Gln Asp Pro Asp Pro Ser Gln Leu His
   35 40
   Arg Ser Ser Leu Val Lys Asn Leu Gln Asn
   45 50
   Ile Tyr Phe Leu Tyr Glu Gly Asp Pro Val
   55 60
   Thr His Glu Asn Val Lys Ser Val Asp Gln
   65 70
   Leu Arg Ser His Asp Leu Ile Tyr Asn Val
   75 80
   Ser Gly Pro Asn Tyr Asp Lys Leu Lys Thr
   85 90
   Glu Leu Lys Asn Gln Glu Met Ala Thr Leu
   95 100
   Phe Lys Asp Lys Asn Val Asp Ile Tyr Gly
   105 110
   Val Glu Tyr Tyr His Leu Cys Tyr Leu Cys
   115 120
   Glu Asn Ala Glu Arg Ser Ala Cys Ile Tyr
   125 130
   Gly Gly Val Thr Asn His Glu Gly Asn His
   135 140
   Leu Glu Ile Pro Lys Lys Ile Val Val Lys
   145 150
   Val Ser Ile Asp Gly Ile Gln Ser Leu Ser
   155 160
   Phe Asp Ile Glu Thr Asn Lys Lys Met Val
   165 170
   Thr Ala Gln Glu Leu Asp Tyr Lys Val Arg
   175 180
   Lys Tyr Leu Thr Asp Asn Lys Gln Leu Tyr
   185 190
   Thr Asn Gly Pro Ser Lys Tyr Glu Thr Gly
   195 200
   Tyr Ile Lys Phe Ile Pro Lys Asn Lys Glu
   205 210
   Ser Phe Trp Phe Asp Phe Phe Pro Glu Pro
                   215                 220
   Glu Phe Thr Gln Ser Lys Tyr Leu Met Ile
                   225                 230
   Tyr Lys Asp Asn Glu Thr Leu Asp Ser Asn
                   235                 240
   Thr Ser Gln Ile Glu Val Tyr Leu Thr Thr
                   245                 250
   Lys
(18) INFORMATION FOR SEQUENCE ID NO:17:
   (i) SEQUENCE CHARACTERISTICS:
      (A) LENGTH: 82
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:17:
   Ser His Asp Gln Phe Leu Gln His Thr Ile
                   5                   10
   Leu Phe Lys Gly Phe Phe Thr Asp His Ser
                   15                  20
   Trp Tyr Asn Asp Leu Leu Val Asp Phe Asp
                   25                  30
   Ser Lys Asp Ile Val Asp Lys Tyr Lys Gly
                   35                  40
   Lys Lys Val Asp Leu Tyr Gly Ala Tyr Tyr
                   45                  50
   Gly Tyr Gln Cys Ala Gly Gly Thr Pro Asn
                   55                  60
   Lys Thr Ala Cys Met Tyr Gly Gly Val Thr
                   65                  70
   Leu His Asp Asn Asn Arg Leu Thr Glu Glu
                   75                  80
   Lys Lys
(19) INFORMATION FOR SEQUENCE ID NO:18:
   (i) SEQUENCE CHARACTERISTICS:
      (A) LENGTH: 82
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:18:
   Thr Gly Asp Gln Phe Leu Glu Asn Thr Leu
                    5                  10
   Leu Tyr Lys Lys Phe Phe Thr Asp Leu Ile
                   15                  20
   Asn Phe Glu Asp Leu Leu Ile Asn Phe Asn
                   25                  30
   Ser Lys Glu Met Ala Gln His Phe Lys Ser
                   35                  40
   Lys Asn Val Asp Val Tyr Pro Ile Arg Tyr
                   45                  50
   Ser Ile Asn Cys Tyr Gly Gly Glu Ile Asp
                   55                  60
   Arg Thr Ala Cys Thr Tyr Gly Gly Val Thr
                   65                  70
   Pro His Glu Gly Asn Lys Leu Lys Glu Arg
                   75                  80
   Lys Lys
(20) INFORMATION FOR SEQUENCE ID NO:19:
   (i) SEQUENCE CHARACTERISTICS:
      (A) LENGTH: 82
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:19:
   Ser Asp Asp Gln Phe Leu Glu Asn Thr Leu
                    5                  10
   Leu Phe Lys Gly Phe Phe Thr Gly His Pro
                   15                  20
   Trp Tyr Asn Asp Leu Leu Val Asp Leu Gly
                   25                  30
   Ser Lys Asp Ala Thr Asn Lys Tyr Lys Gly
                   35                  40
   Lys Lys Val Asp Leu Tyr Gly Ala Tyr Tyr
                   45                  50
   Gly Tyr Gln Cys Ala Gly Gly Thr Pro Asn
                   55                  60
   Lys Thr Ala Cys Met Tyr Gly Gly Val Thr
                   65                  70
   Leu His Asp Asn Asn Arg Leu Thr Glu Glu
                   75                  80
   Lys Lys
(21) INFORMATION FOR SEQUENCE ID NO:20:
   (i) SEQUENCE CHARACTERISTICS:
      (A) LENGTH: 89
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:20:
   Ser Ile Asp Gln Phe Leu Tyr Phe Asp Leu
                    5                   10
   Ile Tyr Ser Ile Lys Asp Thr Lys Leu Gly
                   15                   20
   Asn Tyr Asp Asn Val Arg Val Glu Phe Lys
                   25                   30
   Asn Lys Asp Leu Ala Asp Lys Tyr Lys Asp
                   35                   40
   Lys Tyr Val Asp Val Phe Gly Ala Asn Tyr
                   45                   50
   Tyr Gln Cys Tyr Phe Ser Lys Lys Thr Asn
                   55                   60
   Asp Ile Asn Ser His Gln Thr Asp Lys Arg
                   65                   70
   Lys Thr Cys Met Tyr Gly Gly Val Thr Glu
                   75                   80
   His Asn Gly Asn Gln Leu Asp Lys Tyr
                   85
(22) INFORMATION FOR SEQUENCE ID NO:21:
   (i) SEQUENCE CHARACTERISTICS:
      (A) LENGTH: 89
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:21:
   Ser Val Asp Lys Phe Leu Ala His Asp Leu
                    5                   10
   Ile Tyr Asn Ile Ser Asp Lys Lys Leu Lys
                   15                   20
   Asn Tyr Asp Lys Val Lys Thr Glu Leu Leu
                   25                   30
   Asn Glu Gly Leu Ala Lys Lys Tyr Lys Asp
                   35                   40
   Glu Val Val Asp Val Tyr Gly Ser Asn Tyr
                   45                   50
   Tyr Val Asn Cys Tyr Phe Ser Ser Lys Asp
                   55                   60
   Asn Val Gly Lys Val Thr Gly Gly Lys Thr
                   65                   70
   Cys Met Tyr Gly Gly Ile Thr Lys His Glu
                   75                   80
   Gly Asn His Phe Asp Asn Gly Asn Leu
                   85
(23) INFORMATION FOR SEQUENCE ID NO:22:
   (i) SEQUENCE CHARACTERISTICS:
      (A) LENGTH: 89
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:22:
   Ser Val Asp Lys Phe Leu Ala His Asp Leu
                    5                  10
   Ile Tyr Asn Ile Ser Asp Lys Lys Leu Lys
                   15                  20
   Asn Tyr Asp Lys Val Lys Thr Glu Leu Leu
                   25                  30
   Asn Glu Asp Leu Ala Lys Lys Tyr Lys Asp
                   35                  40
   Glu Val Val Asp Val Tyr Gly Ser Asn Tyr
                   45                  50
   Tyr Val Asn Cys Tyr Phe Ser Ser Lys Asp
                   55                  60
   Asn Val Gly Lys Val Thr Gly Gly Lys Thr
                   65                  70
   Cys Met Tyr Gly Gly Ile Thr Lys His Glu
                   75                  80
   Gly Asn His Phe Asp Asn Gly Asn Leu
                   85
(24) INFORMATION FOR SEQUENCE ID NO:23:
   (i) SEQUENCE CHARACTERISTICS:
      (A) LENGTH:89
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:23:
   Ser Val Asp Lys Phe Leu Ala His Asp Leu
                    5                  10
   Ile Tyr Asn Ile Ser Asp Lys Lys Leu Lys
                   15                  20
   Asn Tyr Asp Lys Val Lys Thr Glu Leu Leu
                   25                  30
   Asn Glu Asp Leu Ala Lys Lys Tyr Lys Asp
                   35                  40
   Glu Val Val Asp Val Tyr Gly Ser Asn Tyr
                   45                  50
   Tyr Val Asn Cys Tyr Phe Ser Ser Lys Asp
                   55                  60
   Asn Val Gly Lys Val Thr Gly Gly Lys Thr
                   65                  70
   Cys Met Tyr Gly Gly Ile Thr Lys His Glu
                   75                  80
   Gly Asn His Phe Asp Asn Gly Asn Leu
                   85
(25) INFORMATION FOR SEQUENCE ID NO:24:
   (i) SEQUENCE CHARACTERISTICS:
      (A) LENGTH: 79
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:24:
   Ser Val Asp Gln Leu Leu Ser His Asp Leu
                   5                   10
   Ile Tyr Asn Val Ser Gly Pro Asn Tyr Asp
                   15                  20
   Lys Leu Lys Thr Glu Leu Lys Asn Gln Glu
                   25                  30
   Met Ala Thr Leu Phe Lys Asp Lys Asn Val
                   35                  40
   Asp Ile Tyr Gly Val Glu Tyr Tyr His Leu
                   45                  50
   Cys Tyr Leu Cys Glu Asn Ala Glu Arg Ser
                   55                  60
   Ala Cys Ile Tyr Gly Gly Val Thr Asn His
                   65                  70
   Glu Gly Asn His Leu Glu Ile Pro Lys
                   75
(26) INFORMATION FOR SEQUENCE ID NO:25:
   (i) SEQUENCE CHARACTERISTICS:
      (A) LENGTH: 73
      (B) TYPE: Amino Acid
      (C) STRANDEDNESS: Unknown
      (D) TOPOLOGY: Unknown
   (ii) Molecule type: Peptide
   (xi) SEQUENCE DESCRIPTION: SEQ ID NO:25: